(19) **Europäisches Patentamt / European Patent Office / Office européen des brevets**

(11) **EP 4 768 505 A1**

(12) **EUROPEAN PATENT APPLICATION**
published in accordance with Art. 153(4) EPC

(43) Date of publication:
01.07.2026 Bulletin 2026/27

(21) Application number: 24870926.3

(22) Date of filing: 27.09.2024

(51) International Patent Classification (IPC):
C07K 16/28 (2006.01)    C07K 19/00 (2006.01)
C12N 15/62 (2006.01)    C12N 5/10 (2006.01)
A61K 39/00 (2006.01)    A61P 35/00 (2006.01)

(52) Cooperative Patent Classification (CPC):
A61K 39/00; A61P 35/00; C07K 16/28;
C07K 19/00; C12N 5/10; C12N 15/62

(86) International application number:
PCT/CN2024/121623

(87) International publication number:
WO 2025/067388 (03.04.2025 Gazette 2025/14)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.09.2023 CN 202311276094

(71) Applicant: Shenzhen Genocury Biotech Co., Ltd
Shenzhen, Guangdong 518000 (CN)

(72) Inventors:
• HUANG, Ke
  Shenzhen, Guangdong 518110 (CN)
• LI, Yuhang
  Shenzhen, Guangdong 518110 (CN)

(74) Representative: Vogelbruch, Keang
VOGELBRUCH Patentanwaltsgesellschaft mbH
Paul-Gerhardt-Straße 16
40593 Düsseldorf (DE)

Remarks:
The complete document including Reference
Table(s) and the Sequence Listing(s) can be
downloaded from the EPO website

(54) **SINGLE-DOMAIN ANTIBODY TARGETING CD79B, CHIMERIC ANTIGEN RECEPTOR, AND USE THEREOF**

(57) A single-domain antibody targeting CD79B, a chimeric antigen receptor, and a use thereof. Provided are an anti-CD79B single-domain antibody, a chimeric antigen receptor constructed using the single-domain antibody, and an engineered immune effector cell. Also provided is a use of the single-domain antibody, the chimeric antigen receptor, and the engineered immune effector cell in the preparation of a drug for the treatment of CD79B and/or B cell-related diseases.

FIG. 1

EP 4 768 505 A1

## Description

### TECHNICAL FIELD

The present invention relates to the technical field of immunotherapy, and in particular, to a single-domain antibody targeting CD79B, a chimeric antigen receptor, and a use thereof.

### BACKGROUND ART

CD79B is part of the B-cell receptor (BCR) signaling complex and is a critical cell surface receptor for the successful development and maintenance of mature B cells. CD79B expression is generally restricted to the B-cell lineage and remains high in most non-Hodgkin lymphoma subtypes, making it one of the ideal tumor antigens for immunotherapy targeting B-cell-related tumors.

Unlike conventional 4-chain antibodies, single-domain antibodies (sdAbs) have a single variable domain of a single-chain antibody. For example, camelids and sharks produce antibodies that are naturally devoid of light chains, which are known as heavy-chain-only antibodies (hcAbs, or simply heavy-chain antibodies). Antigen-binding fragments in each arm of camelid heavy-chain-only antibodies have a single heavy-chain variable domain (VHH) that can have high affinity for antigen without the aid of a light chain. Camelid VHH antibodies are considered the smallest functional antigen-binding fragments, with a molecular weight of only about 15 kDa, and thus are also known as nanobodies. VHH antibodies possess natural advantages of good solubility, high stability, strong penetrability, and broad epitope binding. Since their discovery, VHH antibodies have gradually attracted attention of researchers in the field, and fundamental research on them has become increasingly sophisticated. In terms of application, they have progressively entered the clinical research stage for autoimmune diseases, hematological diseases, viral infections, and orthopedic diseases, among others, and have also demonstrated significant advantages in combating infectious diseases, inflammatory diseases, and neurodegenerative diseases.

Chimeric antigen receptor (CAR)-modified T cells (CAR-T cells), as an immunotherapeutic strategy, have received widespread attention and application in tumor treatment, particularly in hematological malignancies. The principle of CAR-T cell preparation involves genetically modifying T cells to express a CAR structure that can specifically recognize tumor cell surface antigens, and upon specific binding of the CAR to the tumor cell surface antigen, activating its downstream immune co-stimulatory factors and T cells, thereby activating the T cells to secrete relevant cytokines and specifically kill tumor cells. CAR structures typically consist of four parts: an extracellular antigen-binding region (often an antibody with an antigen recognition function, such as a single-chain antibody), a hinge region, a transmembrane region, and an intracellular signaling domain. Currently, based on the number of co-stimulatory molecules incorporated into the intracellular signaling domain, CAR structures are generally classified into first-generation (without co-stimulatory molecule), second-generation (containing one type of co-stimulatory molecule), and third-generation (containing two types of co-stimulatory molecules). The second-generation CAR structure is currently the most widely used in marketed products and clinical research.

Because CAR-T cell manufacturing technology requires the use of single-chain antibodies with good binding activity and efficient epitope binding, one of the key technical aspects of CAR-T cell therapy lies in screening for high-affinity antibodies with good specificity, strong binding ability, and efficient epitope binding. However, the conventional anti-CD79B antibodies, such as monoclonal anti-CD79B antibodies or anti-CD79B single-chain variable region fragments (scFv), are limited by disadvantages such as large molecular weight, weak binding ability to antigens, relatively low affinity, relatively high immunogenicity, difficulty in modification, and poor stability, and require further modification.

Therefore, there remains a widespread need to develop improved anti-CD79B single-domain antibodies, CD79B-targeting CAR structures built upon them, and engineered immune effector cells expressing the CAR structures. For example, there is a need to develop stable, high-affinity, small-sized, and less immunogenic anti-CD79B single-domain antibodies for more effective and efficient CAR-T cell therapy.

### SUMMARY

Accordingly, to address at least one of the above technical problems, one aspect of the present invention provides an anti-CD79B single-domain antibody, wherein the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to an amino acid sequence as set forth in SEQ ID NO:3, SEQ ID NO:11, SEQ ID NO:19, SEQ ID NO:24, or SEQ ID NO:29; the CDR-H2 comprises an amino acid sequence having at least about 75%, 80%, 85%, 90%, 91%, 92%, 93%, 94%, 95%, 96%, 97%, 98%, 99%, 99.5%, or 100% identity to an amino acid sequence as set forth in SEQ ID NO:5, SEQ ID NO:13, or SEQ ID NO:20; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to an amino acid sequence as set forth in SEQ ID NO:7, SEQ ID NO:15, SEQ ID NO:22, SEQ ID NO:26, or SEQ ID NO:31.

In some embodiments of the present invention, the anti-CD79B single-domain antibody comprises CDR-H1, CDR-H2, and CDR-H3;

wherein the CDR-H1 comprises the amino acid sequence as set forth in SEQ ID NO:3, the CDR-H2 comprises the amino acid sequence as set forth in SEQ ID NO:5, and the CDR-H3 comprises the amino acid sequence as set forth in SEQ ID NO:7; or

wherein the CDR-H1 comprises the amino acid sequence as set forth in SEQ ID NO:11, the CDR-H2 comprises the amino acid sequence as set forth in SEQ ID NO:13, and the CDR-H3 comprises the amino acid sequence as set forth in SEQ ID NO:15; or

wherein the CDR-H1 comprises the amino acid sequence as set forth in SEQ ID NO:19, the CDR-H2 comprises the amino acid sequence as set forth in SEQ ID NO:20, and the CDR-H3 comprises the amino acid sequence as set forth in SEQ ID NO:22; or

wherein the CDR-H1 comprises the amino acid sequence as set forth in SEQ ID NO:24, the CDR-H2 comprises the amino acid sequence as set forth in SEQ ID NO:20, and the CDR-H3 comprises the amino acid sequence as set forth in SEQ ID NO:26; or

wherein the CDR-H1 comprises the amino acid sequence as set forth in SEQ ID NO:29, the CDR-H2 comprises the amino acid sequence as set forth in SEQ ID NO:20, and the CDR-H3 comprises the amino acid sequence as set forth in SEQ ID NO:31.

In some embodiments of the present invention, the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:3; the CDR-H2 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:5; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:7.

In some embodiments of the present invention, the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:11; the CDR-H2 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:13; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:15.

In some embodiments of the present invention, the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:19; the CDR-H2 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:20; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:22.

In some embodiments of the present invention, the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:24; the CDR-H2 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:20; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:26.

In some embodiments of the present invention, the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:29; the CDR-H2 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:20; and the CDR-H3 comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:31.

In some embodiments of the present invention, the anti-CD79B single-domain antibody comprises CDR-H1, CDR-H2, and CDR-H3; or

wherein the amino acid sequence of the CDR-H1 is set forth in SEQ ID NO:3, the amino acid sequence of the CDR-H2 is set forth in SEQ ID NO:5, and the amino acid sequence of the CDR-H3 is set forth in SEQ ID NO:7; or wherein the amino acid sequence of the CDR-H1 is set forth in SEQ ID NO:11, the amino acid sequence of the CDR-H2 is set forth in SEQ ID NO:13, and the amino acid sequence of the CDR-H3 is set forth in SEQ ID NO:15; or wherein the amino acid sequence of the CDR-H1 is set forth in SEQ ID NO:19, the amino acid sequence of the CDR-H2 is set forth in SEQ ID NO:20, and the amino acid sequence of the CDR-H3 is set forth in SEQ ID NO:22; or wherein the amino acid sequence of the CDR-H1 is set forth in SEQ ID NO:24, the amino acid sequence of the CDR-H2 is set forth in SEQ ID NO:20, and the amino acid sequence of the CDR-H3 is set forth in SEQ ID NO:26; or wherein the amino acid sequence of the CDR-H1 is set forth in SEQ ID NO:29, the amino acid sequence of the CDR-H2 is set forth in SEQ ID NO:20, and the amino acid sequence of the CDR-H3 is set forth in SEQ ID NO:31.

In some embodiments of the present invention, the anti-CD79B single-domain antibody comprises CDR-H1, CDR-H2, and CDR-H3, wherein the CDR-H1, the CDR-H2, and the CDR-H3 are defined according to the IMGT numbering scheme, the Kabat numbering scheme, the Martin numbering scheme, the AbM numbering scheme, the Chothia numbering scheme, or the Contact numbering scheme.

In some embodiments of the present invention, the CDR-H1, the CDR-H2, and the CDR-H3 are defined according to the Martin numbering scheme.

In some embodiments of the present invention, the anti-CD79B single-domain antibody further comprises FR1, FR2, FR3, and FR4, wherein the FR1 comprises an amino acid sequence as set forth in SEQ ID NO:2, SEQ ID NO:10, SEQ ID NO:18, or SEQ ID NO:28; the FR2 comprises an amino acid sequence as set forth in SEQ ID NO:4 or SEQ ID NO:12; the FR3 comprises an amino acid sequence as set forth in SEQ ID NO:6, SEQ ID NO:14, SEQ ID NO:21, SEQ ID NO:25, or SEQ ID NO:30; and the FR4 comprises an amino acid sequence as set forth in SEQ ID NO:8 or SEQ ID NO:16.

In some embodiments of the present invention, the anti-CD79B single-domain antibody further comprises FR1, FR2, FR3, and FR4, wherein the FR1 comprises the amino acid sequence as set forth in SEQ ID NO:2, the FR2 comprises the amino acid sequence as set forth in SEQ ID NO:4, the FR3 comprises the amino acid sequence as set forth in SEQ ID NO:6, and the FR4 comprises the amino acid sequence as set forth in SEQ ID NO:8.

In some embodiments of the present invention, the anti-CD79B single-domain antibody further comprises FR1, FR2, FR3, and FR4, wherein the FR1 comprises the amino acid sequence as set forth in SEQ ID NO:10, the FR2 comprises the amino acid sequence as set forth in SEQ ID NO:12, the FR3 comprises the amino acid sequence as set forth in SEQ ID NO:14, and the FR4 comprises the amino acid sequence as set forth in SEQ ID NO:16.

In some embodiments of the present invention, the anti-CD79B single-domain antibody further comprises FR1, FR2, FR3, and FR4;

wherein the FR1 comprises the amino acid sequence as set forth in SEQ ID NO:2, the FR2 comprises the amino acid sequence as set forth in SEQ ID NO:4, the FR3 comprises the amino acid sequence as set forth in SEQ ID NO:6, and the FR4 comprises the amino acid sequence as set forth in SEQ ID NO:8; or wherein the FR1 comprises the amino acid sequence as set forth in SEQ ID NO:10, the FR2 comprises the amino acid sequence as set forth in SEQ ID NO:12, the FR3 comprises the amino acid sequence as set forth in SEQ ID NO:14, and the FR4 comprises the amino acid sequence as set forth in SEQ ID NO:16; or wherein the FR1 comprises the amino acid sequence as set forth in SEQ ID NO:18, the FR2 comprises the amino acid sequence as set forth in SEQ ID NO:12, the FR3 comprises the amino acid sequence as set forth in SEQ ID NO:21, and the FR4 comprises the amino acid sequence as set forth in SEQ ID NO:16; or wherein the FR1 comprises the amino acid sequence as set forth in SEQ ID NO:10, the FR2 comprises the amino acid sequence as set forth in SEQ ID NO:12, the FR3 comprises the amino acid sequence as set forth in SEQ ID NO:25, and the FR4 comprises the amino acid sequence as set forth in SEQ ID NO:16; or wherein the FR1 comprises the amino acid sequence as set forth in SEQ ID NO:28, the FR2 comprises the amino acid sequence as set forth in SEQ ID NO:12, the FR3 comprises the amino acid sequence as set forth in SEQ ID NO:30, and the FR4 comprises the amino acid sequence as set forth in SEQ ID NO:16.

In some embodiments of the present invention, the anti-CD79B single-domain antibody further comprises FR1, FR2, FR3, and FR4, wherein the FR1 comprises the amino acid sequence as set forth in SEQ ID NO:18, the FR2 comprises the amino acid sequence as set forth in SEQ ID NO:12, the FR3 comprises the amino acid sequence as set forth in SEQ ID NO:21, and the FR4 comprises the amino acid sequence as set forth in SEQ ID NO:16.

In some embodiments of the present invention, the anti-CD79B single-domain antibody further comprises FR1, FR2, FR3, and FR4, wherein the FR1 comprises the amino acid sequence as set forth in SEQ ID NO:10, the FR2 comprises the amino acid sequence as set forth in SEQ ID NO:12, the FR3 comprises the amino acid sequence as set forth in SEQ ID NO:25, and the FR4 comprises the amino acid sequence as set forth in SEQ ID NO:16.

In some embodiments of the present invention, the anti-CD79B single-domain antibody further comprises FR1, FR2, FR3, and FR4, wherein the FR1 comprises the amino acid sequence as set forth in SEQ ID NO:28, the FR2 comprises the amino acid sequence as set forth in SEQ ID NO:12, the FR3 comprises the amino acid sequence as set forth in SEQ ID NO:30, and the FR4 comprises the amino acid sequence as set forth in SEQ ID NO:16.

In some embodiments of the present invention, the anti-CD79B single-domain antibody comprises the amino acid sequence as set forth in SEQ ID NO:1, SEQ ID NO:9, SEQ ID NO:17, SEQ ID NO:23 or SEQ ID NO:27, or an amino acid sequence having at least about 75%, 80%, 85%, 90%, 91%, 92%, 93%, 94%, 95%, 96%, 97%, 98%, 99% or 99.5% identity to the amino acid sequence as set forth in SEQ ID NO:1, SEQ ID NO:9, SEQ ID NO:17, SEQ ID NO:23 or SEQ ID NO:27.

In some embodiments of the present invention, the amino acid sequence of the anti-CD79B single-domain antibody is as set forth in SEQ ID NO:1, SEQ ID NO:9, SEQ ID NO:17, SEQ ID NO:23 or SEQ ID NO:27.

In some embodiments of the present invention, the anti-CD79B single-domain antibody can specifically bind to the human CD79B protein.

In some embodiments of the present invention, the amino acid sequence of the anti-CD79B single-domain antibody and the amino acid sequences of its CDR-H1, CDR-H2 and CDR-H3 are shown in Table 1 below.

Table 1

| SEQ ID NO:1 | DVQLQESGGGLVQPGGSLRLSCAASGFAFSLYAMSWARQAPGKG LEWVSDIYGDDDSRRNYADSVKGRFTISRDNAKNTVYLQMTNLQ PEDTALYYCGKGRDGTRWSAREYEYRGQGTQVTVSS |
| --- | --- |
| SEQ ID NO:3 | GFAFSLYAMS |
| SEQ ID NO:5 | DIYGDDDSRRN |
| SEQ ID NO:7 | KGRDGTRWSAREYEY |
| SEQ ID N0:9 | DVQLQESGGGLVQPGGSLSLSCAASISSSSIWVRAWHRQAPGKQ RELVAAINNDGQTMYADSVKGRFTISRDNAKTTVSLQMNSLKPE DTAVYYCNVVTPKGHQYWGQGTQVTVSS |
| SEQ ID NO:11 | ISSSSIWVRA |
| SEQ ID NO:13 | AINNDGQTM |
| SEQ ID NO:15 | VVTPKGHQY |
| SEQ ID NO:17 | DVQLQESGGGLMQPGGSLSLTCTASISSLSIWVRAWHRQAPGKQ RELVAAINSDGQTMYADSVKGRFTISRDSAKTTVSLQMDSLKPE DTAVYYCNVVTPKGQQYWGQGTQVTVSS |
| | |
| SEQ ID NO:19 | ISSLSTWVRA |
| SEQ ID NO:20 | AINSDGQTM |
| SEQ ID NO:22 | VVTPKGQQY |
| SEQ ID NO:23 | DVQLQESGGGLVQPGGSLSLSCAASMSSPSLWVRAWHRQAPGKQ RELVAAINSDGQTMYADSVKGRFTISRDNAKTLVSLQMDSLKPE DTAVYYCNVVTPRGQQYWGQGTQVTVSS |
| SEQ ID N0:24 | MSSPSLWVRA |
| SEQ ID NO:26 | VVTPRGQQY |

(continued)

| SEQ ID NO:27 | DVQLQESGGGLVQPGGSLSLTCTASISSLTIWVRAWHRQAPGKQ RELVAAINSDGQTMYADSVKGRFTISRDNAKTTVSLQMDSLKPE DTAVYYCNVVTPNGQQYWGQGTQVTVSS |
|---|---|
| SEQ ID NO:29 | ISSLTIWVRA |
| SEQ ID NO:31 | VVTPNGQQY |

In another aspect, the present invention discloses an isolated nucleic acid encoding any one of the foregoing anti-CD79B single-domain antibodies.

In another aspect, the present invention discloses a chimeric antigen receptor (CAR), wherein the chimeric antigen receptor comprises:

(a) an extracellular antigen-binding region;
(b) a transmembrane region; and
(c) an intracellular signaling domain;

wherein the extracellular antigen-binding region comprises any one of the foregoing anti-CD79B single-domain antibodies.

In some embodiments of the present invention, the transmembrane region of the CAR is derived from a transmembrane region of at least one of the following proteins: CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcεRIγ, IL7, IL12, IL15, SLAM, KIR2DL4, KIR2DS1, KIR2DS2, NKG2C, NKG2D, and CS1;
preferably, the transmembrane region of the CAR is derived from the transmembrane region of CD8α.

In some embodiments of the present invention, the transmembrane region of the CAR is derived from the transmembrane region of CD8α.

In some embodiments of the present invention, the amino acid sequence of the transmembrane region of CD8α has at least about 75%, 80%, 85%, 90%, 91%, 92%, 93%, 94%, 95%, 96%, 97%, 98%, 99%, 99.5%, or 100% identity to SEQ ID NO:34.

In some embodiments of the present invention, the intracellular signaling domain of the CAR is derived from an intracellular signaling domain of at least one of the following proteins: CD3ε, CD3γ, CD3δ, CD3ζ, CD79a, CD79B, FcεRIγ, FcεRβ, FcγRIIa, bovine leukemia virus gp30, Epstein-Barr virus (EBV) LMP2A, simian immunodeficiency virus PBj14 Nef, DAP10, DAP12, and intracellular signaling domains of other proteins whose intracellular signaling domain comprises at least one ITAM;
preferably, the intracellular signaling domain of the CAR is derived from the intracellular signaling domain of CD3ζ.

In some embodiments of the present invention, the intracellular signaling domain of the CAR is derived from the intracellular signaling domain of CD3ζ.

In some embodiments of the present invention, the amino acid sequence of the intracellular signaling domain of CD3ζ has at least about 75%, 80%, 85%, 90%, 91%, 92%, 93%, 94%, 95%, 96%, 97%, 98%, 99%, 99.5%, or 100% identity to SEQ ID NO:36.

In some embodiments of the present invention, the chimeric antigen receptor further comprises a hinge region linking the extracellular antigen-binding region and the transmembrane region;

preferably, the hinge region is derived from a hinge region of at least one of the following proteins: CD28, CD8, CD8α, CD8β, CD3, CD45, Ig4, CD4, CD5, CD8, CD9, CD16, CD22, CD33, CD37, CD134, CD137, ICOS, and CD154;
more preferably, the hinge region is derived from the hinge region of CD8α.

In some embodiments of the present invention, the hinge region of the CAR links the extracellular antigen-binding region and the transmembrane region, and the hinge region is located between C-terminus of the extracellular antigen-binding region and N-terminus of the transmembrane region.

In some embodiments of the present invention, the hinge region of the CAR is derived from the hinge region of CD8α.

In some embodiments of the present invention, an amino acid sequence of the hinge region of CD8α has at least about 75%, 80%, 85%, 90%, 91%, 92%, 93%, 94%, 95%, 96%, 97%, 98%, 99%, 99.5%, or 100% identity to SEQ ID NO:33.

In some embodiments of the present invention, the chimeric antigen receptor further comprises a costimulatory signaling domain;

preferably, the costimulatory signaling domain comprises the costimulatory signaling domain of at least one of the following proteins: CD28, 4-1BB, CD27, CD2, CD7, CD8, CD8α, CD8β, OX40, CD226, DR3, SLAM, CDS, ICAM-1, NKG2D, NKG2C, B7-H3, 2B4, FcαRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88;
more preferably, the costimulatory signaling domain comprises the costimulatory signaling domain of 4-1BB.

In some embodiments of the present invention, the costimulatory signaling domain of the CAR is the costimulatory signaling domain of 4-1BB.

In some embodiments of the present invention, an amino acid sequence of the costimulatory signaling domain of 4-1BB has at least about 75%, 80%, 85%, 90%, 91%, 92%, 93%, 94%, 95%, 96%, 97%, 98%, 99%, 99.5%, or 100% identity to SEQ ID NO:35.

In some embodiments of the present invention, the chimeric antigen receptor further comprises a signal peptide at N-terminus of the chimeric antigen receptor;

preferably, the signal peptide is derived from the signal peptide of at least one of the following proteins: HLA-A, CD8α, CD33, Igκ, IL-2, and GM-CSFRα;
more preferably, the signal peptide is derived from the signal peptide of CD8α.

In some embodiments of the present invention, the signal peptide is derived from the signal peptide of CD8α.

In some embodiments of the present invention, the amino acid sequence of the signal peptide of CD8α has at least about 75%, 80%, 85%, 90%, 91%, 92%, 93%, 94%, 95%, 96%, 97%, 98%, 99%, 99.5%, or 100% identity to SEQ ID NO:32.

In some embodiments of the present invention, the CAR can specifically bind to the human CD79B protein.

In another aspect, the present invention discloses an isolated nucleic acid encoding any one of the foregoing chimeric antigen receptors.

In some embodiments of the present invention, the nucleic acid further comprises a nucleic acid encoding a signal peptide, wherein the signal peptide is located at N-terminus of the chimeric antigen receptor.

In some embodiments of the present invention, the signal peptide is derived from the signal peptide of CD8α.

In another aspect, the present invention discloses a vector, wherein the vector comprises the isolated nucleic acid encoding any one of the foregoing chimeric antigen receptors.

In some embodiments of the present invention, the vector is at least one selected from the group consisting of lipid nanoparticles, virus-like particles, adenovirus, adeno-associated virus, lentiviral vector, and retroviral vector;
preferably, the vector is a lentiviral vector or a retroviral vector.

In some embodiments of the present invention, the vector is a lentiviral vector or a retroviral vector.

In another aspect, the present invention discloses a targeting vector, wherein a surface of the targeting vector comprises a targeting molecule, and the targeting molecule comprises any one of the foregoing anti-CD79B single-domain antibodies disclosed in the present invention.

In some embodiments of the present invention, the targeting vector is a lentiviral vector or a retroviral vector, the targeting molecule further comprises a transmembrane region, and the anti-CD79B single-domain antibody is directly or indirectly linked to the transmembrane region and is exposed on the surface of the lentiviral vector or retroviral vector;

preferably, the transmembrane region is derived from the transmembrane region of any one of the following proteins: CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcεRIγ, IL7, IL12, IL15, SLAM, KIR2DL4, KIR2DS1, KIR2DS2, NKG2C, NKG2D, and CS1;
more preferably, the transmembrane region is derived from the transmembrane region of CD8α.

In some embodiments of the present invention, the transmembrane region of the targeting molecule is the transmembrane region of CD8α; preferably, the amino acid sequence of the transmembrane region of CD8α has at least about 75%, 80%, 85%, 90%, 91%, 92%, 93%, 94%, 95%, 96%, 97%, 98%, 99%, 99.5%, or 100% identity to SEQ ID NO:34.

In some embodiments of the present invention, the targeting molecule further comprises a linking domain, and the anti-CD79B single-domain antibody is indirectly linked to the transmembrane region of the targeting molecule via the linking domain;

preferably, the linking domain is selected from the group consisting of:

(i) an immunoglobulin hinge region, wherein the immunoglobulin hinge region is selected from wild-type or modified hinge regions of IgG1, IgG2, IgG3, IgG4, IgA, and IgD;
(ii) a hinge region, wherein the hinge region is selected from wild-type or modified hinge regions of proteins selected from the group consisting of: CD28, CD7, CD8, CD8α, CD8β, CD3, CD45, CD4, CD5, CD8, CD9, CD16, CD22, CD33, CD37, CD134, CD137, ICOS, and CD154;
(iii) all or part of an Fc domain, wherein the Fc domain is one or more domains selected from the group consisting of CH1 domain, CH2 domain, and CH3 domain; and
(iv) a stalk domain of a type II C-lectin, wherein the type II C-lectin is selected from the stalk domains of CD23, CD69, CD72, CD94, NKG2A, and NKG2D;

more preferably, the linking domain is the hinge region of CD8α.

In some embodiments of the present invention, the linking domain of the targeting molecule is the hinge region of CD8α;
preferably, an amino acid sequence of the hinge region of CD8α has at least about 75%, 80%, 85%, 90%, 91%, 92%, 93%, 94%, 95%, 96%, 97%, 98%, 99%, 99.5%, or 100% identity to SEQ ID NO:33.
In some embodiments of the present invention, the viral envelope of the lentiviral vector or retroviral vector comprises the targeting molecule.
In another aspect, the present invention discloses an engineered effector cell, wherein the engineered effector cell comprises any one of the foregoing chimeric antigen receptors or the nucleic acid encoding any one of the foregoing chimeric antigen receptors;

preferably, the engineered immune effector cell is at least one selected from the group consisting of T cell, B cell, NK cell, macrophage, dendritic cell, and induced pluripotent stem cell;
more preferably, the engineered immune effector cell is an engineered T cell.

In some embodiments of the present invention, the engineered immune effector cell is an engineered T cell, and the engineered T cell expresses the chimeric antigen receptor (CAR-T cell).
In some embodiments of the present invention, the CAR-T cell is prepared *in vivo* in a subject or *in vitro;*

when the CAR-T cell is prepared *in vitro* for a subject, the subject is an individual to whom the CAR-T cell is administered;
when the CAR-T cell is prepared *in vivo* in the subject, the subject is an individual to whom a vector comprising a nucleic acid encoding the CAR is administered.

In another aspect, the present invention discloses a composition, wherein the composition comprises a pharmaceutically acceptable excipient or carrier and any one of the following components: (a) any one of the foregoing anti-CD79B single-domain antibodies, (b) a chimeric antigen receptor, (c) an engineered immune effector cell, (d) a nucleic acid encoding any one of the foregoing anti-CD79B single-domain antibodies, or (e) a nucleic acid encoding any one of the foregoing CARs.
In some embodiments of the present invention, the composition comprises a pharmaceutically acceptable excipient or carrier and any one of the following components: any one of the foregoing anti-CD79B single-domain antibodies, the chimeric antigen receptor, the vector comprising a nucleic acid encoding the CAR, and the engineered immune effector cell.
In another aspect, the present invention discloses a method for treating a disease in a subject, wherein the method comprises administering a therapeutically effective amount of any one of the foregoing anti-CD79B single-domain antibodies, the vector comprising a nucleic acid encoding a CAR, the engineered immune effector cell, or the composition to the subject;

preferably, the disease is a B cell-related disease and/or a CD79B-related disease;
more preferably, the disease is a B cell-related and/or CD79B-related malignancy;
even more preferably, the B cell-related malignancy is B cell leukemia or B cell lymphoma, wherein the B cell leukemia or B cell lymphoma is selected from the group consisting of marginal zone lymphoma, diffuse large B-cell lymphoma, mantle cell lymphoma, primary central nervous system lymphoma, primary mediastinal B-cell lymphoma, small lymphocytic lymphoma, B-cell prolymphocytic leukemia, follicular lymphoma, Burkitt lymphoma, primary intraocular lymphoma, chronic lymphocytic leukemia, acute lymphoblastic leukemia, hairy cell leukemia, precursor B-cell lymphoblastic leukemia, non-Hodgkin lymphoma, high-grade B-cell lymphoma, and multiple myeloma.

In some embodiments of the present invention, the disease is a B cell-related and/or CD79B-related autoimmune and/or inflammatory disease, more specifically wherein the B cell-related autoimmune and/or inflammatory disease is associated with inappropriate or increased B cell number and/or activation.

In another aspect, the present invention discloses use of any one of the foregoing anti-CD79B single-domain antibodies, isolated nucleic acid encoding the anti-CD79B single-domain antibody, chimeric antigen receptor, isolated nucleic acid encoding the CAR, vector, targeting vector, engineered immune effector cell, or composition in the manufacture of a medicament for treating a B cell-related and/or CD79B-related malignancy, a B cell-related and/or CD79B-related autoimmune and/or inflammatory disease;

preferably, the B cell-related malignancy is B cell leukemia or B cell lymphoma, wherein the B cell leukemia or B cell lymphoma is selected from the group consisting of marginal zone lymphoma, diffuse large B-cell lymphoma, mantle cell lymphoma, primary central nervous system lymphoma, primary mediastinal B-cell lymphoma, small lymphocytic lymphoma, B-cell prolymphocytic leukemia, follicular lymphoma, Burkitt lymphoma, primary intraocular lymphoma, chronic lymphocytic leukemia, acute lymphoblastic leukemia, hairy cell leukemia, precursor B-cell lymphoblastic leukemia, non-Hodgkin lymphoma, high-grade B-cell lymphoma, and multiple myeloma.

In another aspect, the present invention discloses a reagent for detecting a CD79B protein or a functional domain comprising epitopes thereof, wherein the reagent comprises any one of the foregoing anti-CD79B single-domain antibodies;

preferably, the CD79B protein is a human CD79B protein;

more preferably, the reagent further comprises a modification moiety linked to the anti-CD79B single-domain antibody, wherein the modification moiety comprises a detectable label or a therapeutic agent;

even more preferably, the detectable label comprises at least one selected from the group consisting of an enzyme, a tag protein, a radionuclide, a fluorescent dye, a luminescent substance, and biotin.

In another aspect, the present invention discloses a method for detecting a CD79B protein or a functional domain comprising epitopes thereof for non-diagnostic purposes, wherein the method comprises obtaining a sample suspected of containing the CD79B protein or the functional domain comprising the epitopes thereof, contacting any one of the foregoing anti-CD79B single-domain antibodies or the reagent with the sample to form an antibody-antigen complex, and detecting the presence of the antibody-antigen complex;

preferably, the CD79B protein is a human CD79B protein.

Beneficial effects of the present invention include the following:

The anti-CD79B single-domain antibody provided in the present invention has the advantages of small molecular weight, high solubility, high stability, low immunogenicity, and high tissue penetration, while requiring no additional folding and assembly steps or linker optimization and modification, making it a promising alternative to scFv single-chain antibodies with relatively large molecular weights. Furthermore, CAR-T cells prepared using the chimeric antigen receptor constructed with the anti-CD79B single-domain antibody exhibit significant and efficient killing ability against CD79B$^+$ tumor cells.

Terminology Explanation:

As used in the present invention, the term "antibody" includes monoclonal antibodies (including full-length antibodies having an immunoglobulin Fc region), antibody compositions with multi-epitope specificity, multispecific antibodies (e.g., bispecific antibodies), diabodies, single-chain fragment variable (scFv, also known as single-chain antibodies), single-domain antibodies (VHH or nanobodies), and antibody fragments, particularly antigen-binding fragments such as Fab', F(ab'), and F$v_2$. In some embodiments of the present invention, the terms "immunoglobulin (Ig)" and "antibody" are used interchangeably.

The "variable region" or "variable domain" of an antibody refers to an amino-terminal domain of a heavy or light chain of the antibody. The variable domains of the heavy and light chains may be referred to as "VH" and "VL", respectively. These domains are usually the most variable parts of an antibody (relative to other antibodies of the same type) and contain antigen-binding sites.

The basic 4-chain antibody unit is a heterotetrameric glycoprotein composed of two identical light chains (L) and two identical heavy chains (H). An IgM antibody consists of 5 basic heterotetrameric units and an additional polypeptide called a J chain, and contains 10 antigen-binding sites; while IgA antibodies comprise 2-5 basic 4-chain units, which can polymerize to form polyvalent assemblages in combination with the J chain. In the case of IgG, the 4-chain unit is typically about 150,000 Daltons. Each light chain is linked to a heavy chain by one covalent disulfide bond, while two heavy chains are linked to each other by one or more disulfide bonds, the number of which depends on the isotype of the heavy chains.

Each heavy and light chain also has regularly spaced intrachain disulfide bridges. Each heavy chain has a variable domain (VH) at its N-terminus, followed by three (CH1, CH2, and CH3 for each $\alpha$ and $\gamma$ chain) and four (CH1, CH2, CH3, and CH4 for $\mu$ and $\epsilon$ isotypes) constant domains (CH), and a hinge region (Hinge) located between the CH1 domain and the CH2 domain. Each light chain has a variable domain (VL) at its N-terminus, followed by a constant domain (CL) at its other end. VL is aligned with VH, while CL is aligned with the first constant domain (CH1) of the heavy chain. Specific amino acid residues are thought to form interfaces between the light and heavy chain variable domains. The paired VH and VL together form an antigen-binding site. For the structure and properties of different classes of antibodies, see Basic and Clinical Immunology, Eighth edition, Daniel P. Sties, Abba I. Terr and Tristram G. Parsolw, Appleton & Lange, Norwalk, CT. 1994, p. 71 and Chapter 6. Light chains from any vertebrate species can be classified into one of two distinct types, called $\kappa$ and $\lambda$, based on their constant domain amino acid sequences. Immunoglobulins can be classified into different classes or isotypes based on their heavy chain constant domain (CH) amino acid sequences. There are five classes of immunoglobulins: IgA, IgD, IgE, IgG, and IgM, with heavy chains called $\alpha$, $\delta$, $\epsilon$, $\gamma$, and $\mu$, respectively. Based on relatively minor differences in CH sequence and function, the $\gamma$ and $\alpha$ classes can be further subdivided into subclasses; for example, humans express the following subclasses: IgG1, IgG2A, IgG2B, IgG3, IgG4, IgA1, and IgA2.

Heavy chain antibodies are antibodies derived from camelids or cartilaginous fishes. Compared to the 4-chain antibodies mentioned above, the heavy chain antibodies lack the light chain and the heavy chain constant region 1 (CH1), and only contain two heavy chains composed of a variable region (VHH) and other constant regions. The variable region is linked to the constant region via a hinge-like structure. Each heavy chain of camelid heavy chain antibodies contains one variable region (VHH) and two constant regions (CH2 and CH3), while each heavy chain of cartilaginous fish heavy chain antibodies contains one variable region and five constant regions (CH1-CH5). The antigen-binding fragments of heavy chain antibodies include VHH and single-chain heavy chain antibodies. By fusing with the Fc constant region of human IgG, heavy chain antibodies can possess the CH2 and CH3 of the human IgG Fc region.

The terms "anti-CD79B single-domain antibody," "heavy chain variable region domain of anti-CD79B heavy chain antibody," "anti-CD79B VHH," and "anti-CD79B nanobody" in the present invention are used interchangeably and all refer to a single-domain antibody that specifically recognizes and binds to CD79B. A single-domain antibody is a variable region of a heavy chain antibody. Typically, a single-domain antibody contains three CDRs and four FRs. A single-domain antibody is the smallest functional antigen-binding fragment. Typically, an antibody naturally lacking light chains and heavy chain constant region 1 (CH1) is first obtained, and then the variable region of the heavy chain of the antibody is cloned to construct a single-domain antibody consisting solely of one heavy-chain variable region.

Without substantially affecting the antibody activity, those skilled in the art could alter one or more (e.g., 1, 2, 3, 4, 5, 6, 7, 8, 9, 10 or more) amino acids in the sequence of the present invention to obtain variants of the sequence of the antibody or the functional fragment thereof. These variants include, but are not limited to: deletions, insertions, and/or substitutions of one or more (typically 1-50, preferably 1-30, more preferably 1-20, most preferably 1-10) amino acids, as well as additions of one or more (typically within 20, preferably within 10, more preferably within 5) amino acids at C-terminus and/or N-terminus. In the art, conservative substitution using amino acids with similar or comparable properties generally does not alter protein function. For example, amino acids with similar properties in the FR and/or CDR are substituted. Amino acid residues available for conservative substitution are well known in the art, and the amino acid residues substituted by the foregoing methods may or may not be encoded by a genetic code. For another example, adding one or several amino acids at the C-terminus and/or N-terminus generally does not alter the function of the protein. They are all considered to be encompassed within the scope of protection of the present invention.

"Sequence identity": Generally speaking, "sequence identity" or "sequence homology" refers to the exact nucleotide-to-nucleotide or amino acid-to-amino acid correspondence between two polynucleotide or polypeptide sequences. Typically, techniques for determining sequence identity include determining a nucleotide sequence of a polynucleotide and/or an amino acid sequence encoded thereby, and comparing these sequences to a second nucleotide or amino acid sequence. Two or more sequences (polynucleotides or amino acids) can be compared by determining their "percent identity". For both nucleic acid and amino acid sequences, the percent identity between two sequences is calculated by dividing the number of exact matches between the aligned sequences by the length of the shorter sequence, and then multiplying the result by 100. For example, sequence information can also be compared using the advanced BLAST computer program available from the National Institutes of Health, so as to determine the percent identity. The BLAST program is based on the alignment method described in Karlin and Altschul, Proc. Natl. Acad. Sci. USA 87: 2264-2268 (1990), and discussed in Altschul et al., J. Mol. Biol. 215: 403-410 (1990); Karlin and Altschul, Proc. Natl. Acad. Sci. USA 90: 5873-5877 (1993); and Altschul et al., Nucleic Acids Res. 25: 3389-3402 (1997). Briefly, the BLAST program defines identity as the number of identical aligned symbols (usually nucleotides or amino acids) divided by the total number of symbols in the shorter of the two sequences. The program can be used to determine the percent identity across the entire length of the proteins being compared.

In some embodiments of the present invention, the sequences of the variants in the present invention may have at least about 75%, 80%, 85%, 90%, 91%, 92%, 93%, 94%, 95%, 96%, 97%, 98%, 99%, or 99.5% identity to their source sequences. The sequence identity in the present invention can be measured using sequence analysis software, such as the computer program BLAST with default parameters, particularly BLASTP or TBLASTN. The present invention further includes molecules comprising an antibody heavy chain variable region with CDRs, provided that the CDRs thereof have more than 90% (preferably more than 95%, most preferably more than 98%) homology to the CDRs identified herein.

The single-domain antibodies, nanobodies, or heavy-chain antibodies of the present invention can be prepared using conventional methods in the art, such as phage display technology well known in the art; alternatively, various antibodies of the present invention can be expressed in other cell lines. Suitable mammalian host cells can be transformed with sequences encoding various antibodies of the present invention. Transformation can be performed using any known method, including, for example, packaging polynucleotides in a virus (or viral vector) and transducing the host cell with the virus (or vector). The transformation procedure used depends on the host to be transformed. Methods for introducing heterologous polynucleotides into mammalian cells are well known in the art, including dextran-mediated transfection, calcium phosphate precipitation, polybrene-mediated transfection, protoplast fusion, electroporation, encapsulation of polynucleotides in liposomes, and direct microinjection of DNA into nucleus. Host mammalian cell lines suitable for expression are well known in the art, such as various immortalized cell lines available from the American Type Culture Collection (ATCC), including, but not limited to, Chinese hamster ovary (CHO) cells, HeLa cells, baby hamster kidney (BHK) cells, monkey kidney cells (COS), and human hepatocellular carcinoma cells (e.g., HepG2), among others. In particular, preferred cell lines are selected by identifying those that have high expression levels and produce antibodies with basic CD79B-binding properties.

"Specific binding": As used herein, the term "specific binding" refers to binding that occurs between paired molecular species (e.g., receptor and ligand). When the interaction between two species results in the formation of a non-covalently bound complex, binding that occurs is typically the result of electrostatic, hydrogen bonding, or lipophilic interaction. In various embodiments, the specific binding between one or more species is direct. In some embodiments of the present invention, the affinity of specific binding is about 2-fold, about 5-fold, about 10-fold, about 20-fold, about 50-fold, about 100-fold, or about 1000-fold or higher than that of background binding (non-specific binding).

As used in the present invention, the term "chimeric antigen receptor (CAR)" comprises an extracellular antigen-binding region, wherein the extracellular antigen-binding region comprises any one of the anti-CD79B single-domain antibodies (sdAbs) disclosed in the present invention, such as VHH.

In some embodiments of the present invention, the CAR disclosed in the present invention comprises: (a) an extracellular antigen-binding region; (b) a transmembrane region; and (c) an intracellular signaling domain, wherein the extracellular antigen-binding region comprises the anti-CD79B single-domain antibody (sdAb) disclosed in the present invention.

The CAR disclosed in the present invention comprises an extracellular antigen-binding region that comprises one or more sdAbs. The sdAbs may be of identical or different origins and have identical or different sizes. Any sdAb known in the art or disclosed in the present invention, including the single-domain antibodies disclosed in the present invention, may be used for constructing the CAR described herein. The sdAbs may be derived from any species, including, but not limited to, mouse, rat, human, camel, llama, lamprey, shark, goat, rabbit, and bovine. The single-domain antibodies contemplated by the present invention also encompass naturally occurring single-domain antibody molecules from species other than camelids and sharks

In some embodiments of the present invention, the extracellular antigen-binding region disclosed in the present invention comprises at least one binding domain, wherein the at least one binding domain comprises the anti-CD79B single-domain antibody disclosed in the present invention.

In some embodiments of the present invention, the anti-CD79B sdAb is camelid-derived, chimeric, human, or humanized.

In some embodiments of the present invention, the chimeric antigen receptor disclosed in the present invention comprises: (a) an extracellular antigen-binding region, wherein the extracellular antigen-binding region comprises any one of the foregoing anti-CD79B single-domain antibodies; (b) a transmembrane region; and (c) an intracellular signaling domain, wherein the anti-CD79B single-domain antibody comprises an amino acid sequence having at least about 75%, 80%, 85%, 90%, 91%, 92%, 93%, 94%, 95%, 96%, 97%, 98%, 99%, 99.5%, or 100% identity to the amino acid sequence as set forth in SEQ ID NO:1, SEQ ID NO:9, SEQ ID NO:17, SEQ ID NO:23, or SEQ ID NO:27.

In some embodiments of the present invention, the chimeric antigen receptor disclosed in the present invention comprises any one of the foregoing anti-CD79B single-domain antibodies, wherein the anti-CD79B single-domain antibody comprises CDR-H1, CDR-H2, and CDR-H3, wherein the CDR-H1 comprises an amino acid sequence having at least about 75%, 80%, 85%, 90%, 91%, 92%, 93%, 94%, 95%, 96%, 97%, 98%, 99%, 99.5%, or 100% identity to SEQ ID NO:3, SEQ ID NO:11, SEQ ID NO:19, SEQ ID NO:24 or SEQ ID NO:29; the CDR-H2 comprises an amino acid sequence having at least about 75%, 80%, 85%, 90%, 91%, 92%, 93%, 94%, 95%, 96%, 97%, 98%, 99%, 99.5%, or 100% identity to SEQ ID NO:5, SEQ ID NO:13 or SEQ ID NO:20; the CDR-H3 comprises an amino acid sequence having at least about 75%, 80%, 85%, 90%, 91%, 92%, 93%, 94%, 95%, 96%, 97%, 98%, 99%, 99.5%, or 100% identity to SEQ ID NO:7, SEQ ID NO:15, SEQ ID NO:22, SEQ ID NO:26 or SEQ ID NO:31.

In some embodiments of the present invention, the chimeric antigen receptor disclosed in the present invention comprises: (a) an extracellular antigen-binding region, wherein the extracellular antigen-binding region comprises any one of the foregoing anti-CD79B single-domain antibodies; (b) a transmembrane region; and (c) an intracellular signaling domain, wherein the anti-CD79B single-domain antibody comprises the amino acid sequence as set forth in SEQ ID NO:1, SEQ ID NO:9, SEQ ID NO:17, SEQ ID NO:23, or SEQ ID NO:27.

In addition to the extracellular antigen-binding region disclosed in the present invention, the chimeric antigen receptor disclosed in the present invention further may comprise one or more structures selected from the group consisting of: a linker (e.g., a peptide linker, including flexible linkers and rigid linkers), a signal peptide, a hinge region, a transmembrane region, a costimulatory signaling domain, and an intracellular signaling domain.

In some embodiments of the present invention, different domains of the chimeric antigen receptor can also be fused together via peptide linkers. Depending on the structural and/or functional characteristics of the single-domain antibody and/or various domains, each peptide linker in the chimeric antigen receptor may have the same or different lengths and/or sequences. Those skilled in the art could independently select and optimize each peptide linker. In some embodiments of the present invention, a peptide linker contains flexible residues (e.g., glycine and serine) such that adjacent domains can move freely relative to each other. For example, a glycine-serine doublet can be a suitable peptide linker.

The peptide linker may be of any suitable length. In some embodiments of the present invention, the peptide linker is at least about 1, 2, 3, 4, 5, 6, 7, 8, 9, 10, 11, 12, 13, 14, 15, 16, 17, 18, 19, 20, 25, 30, 35, 40, 50, 60, 70, 80, 90, 100 or more amino acids in length.

In some embodiments of the present invention, the peptide linker is no more than about 100, 90, 80, 75, 70, 60, 50, 40, 35, 30, 25, 20, 19, 18, 17, 16, 15, 14, 13, 12, 11, 10, 9, 8, 7, 6, 5 or fewer amino acids in length. In some embodiments of the present invention, the peptide linker is about 1 to about 10 amino acids, about 1 to about 20 amino acids, about 1 to about 30 amino acids, about 5 to about 15 amino acids, about 10 to about 25 amino acids, about 5 to about 30 amino acids, about 10 to about 30 amino acids, about 30 to about 50 amino acids, about 50 to about 100 amino acids, or about 1 to about 100 amino acids in length.

The peptide linker may have a naturally occurring sequence or a non-naturally occurring sequence. For example, a sequence derived from the hinge region of a heavy-chain-only antibody may be used as a linker. See, e.g., WO1996/34103.

In some embodiments of the present invention, the peptide linker is a flexible linker. Exemplary flexible linkers include, but are not limited to, glycine polymers (G)n, glycine-serine polymers (e.g., (GS)n, (GSG)n, (GGGS)n, and (GGGGS)n, wherein n is an integer greater than or equal to 1), glycine-alanine polymers, alanine-serine polymers, and other flexible linkers known in the art.

Flexible linkers: Flexible linkers are usually applied when the linked domains require a certain degree of movement or interaction (Chen X, Zaro JL, Shen WC., Fusion protein linkers: property, design and functionality. Adv Drug Deliv Rev. 2013 Oct; 65(10): 1357-69.). Flexible linkers typically consist of small, non-polar (e.g., Gly) or polar amino acids (e.g., Ser or Thr) (Argos P. An investigation of oligopeptides linking domains in protein tertiary structures and possible candidates for general gene fusion. J Mol Biol. 1990; 211:943-958.). These small-sized amino acids provide flexibility while allowing movement of linked functional domains. For commonly used flexible linkers, reference is made to Chen X, Zaro JL, Shen WC., Fusion protein linkers: property, design and functionality. Adv Drug Deliv Rev. 2013 Oct; 65(10):1357-69, which is incorporated herein by reference in its entirety.

The present invention also discloses nucleic acids encoding any one of the foregoing anti-CD79B single-domain antibodies or chimeric antigen receptors. The nucleic acid/polynucleotide of the present invention can be in the DNA or RNA form. The DNA form includes cDNA, genomic DNA, or artificially synthesized DNA. The DNA can be single-stranded or double-stranded. The DNA can be a coding strand or a non-coding strand.

"Nucleic Acid": It refers to any compound and/or substance comprising a polymer of nucleotides, such as polynucleotides. Each nucleotide consists of a base, particularly a purine or pyrimidine base (i.e., cytosine (C), guanine (G), adenine (A), thymine (T) or uracil (U)), a sugar (i.e., deoxyribose or ribose), and a phosphate group. Typically, a nucleic acid molecule is described by a sequence of bases, whereby the bases represent a primary structure (linear structure) of the nucleic acid molecule. The sequence of bases is usually denoted as 5' to 3' direction. As used herein, the term "nucleic acid" encompasses deoxyribonucleic acid (DNA) including, for example, complementary DNA (cDNA) and genomic DNA, ribonucleic acid (RNA), particularly messenger RNA (mRNA), synthetic forms of DNA or RNA, as well as mixed polymers containing two or more of these molecules. A "nucleic acid" can be linear or circular. In addition, "nucleic acid" includes both sense strand (coding strand) and antisense strand (template strand), as well as single-stranded and double-stranded forms. Furthermore, the "nucleic acid" described herein may contain naturally occurring or non-naturally occurring nucleotides. Examples of non-naturally occurring nucleotides include modified nucleotide bases with derivatized sugar or phosphate backbone linkages or chemically modified residues.

As is well known to those skilled in the art, due to the degeneracy of the genetic code, a vast quantity of nucleic acids can be produced, all of which encode the antibodies or chimeric antigen receptors of the present invention. Therefore, once a particular amino acid sequence has been identified, those skilled in the art could produce any number of different nucleic acids by simply modifying the sequence of one or more codons without altering the amino acid sequence of the encoded protein. Thus, the present invention further relates to polynucleotides that hybridize with the aforementioned polynucleotide sequences and have at least 50%, preferably at least 70%, and more preferably at least 80% identity between two sequences. The present invention particularly relates to polynucleotides that are hybridizable to the polynucleotides described in the present invention under stringent conditions. In the present invention, "stringent conditions" means that: (1) hybridization and elution are carried out at lower ionic strength and higher temperatures, such as $0.2 \times$ SSC, 0.1% Ficoll, 42 °C, etc.; or (3) hybridization only occurs when the identity between two sequences is at least more than 90%, more preferably more than 95%. Furthermore, the polypeptides encoded by hybridizable polynucleotides have the same biological functions and activities as mature polypeptides.

The full-length nucleic acid sequences of various antibodies or chimeric antigen receptors of the present invention or fragments thereof generally can be obtained by PCR amplification method, recombinant method, or artificial synthesis method. A feasible method is to synthesize a relevant sequence artificially, especially when the fragment is relatively short. Typically, long fragments can be obtained by first synthesizing multiple small fragments and then ligating them. Furthermore, a coding sequence of a heavy chain and an expression tag (such as 6His) can also be fused together to form a fusion protein.

Once the relevant sequence is obtained, the relevant sequence can be obtained in large quantities using recombinant methods. This typically involves cloning it into a vector, transferring the vector into a cell, and then isolating the relevant sequence from the proliferated host cells by conventional methods. Biomolecules (nucleic acids, polypeptides, etc.) involved in the present invention include those existing in an isolated form. At present, it is already possible to obtain a DNA sequence encoding the polypeptide of the present invention (or fragments or derivatives thereof) completely through chemical synthesis. The DNA sequence can then be introduced into various existing DNA molecules (or vectors) and cells known in the art. In addition, mutations can also be introduced into the polypeptide sequences of the present invention through chemical synthesis.

The present invention further relates to nucleic acid constructs, such as expression vectors and recombinant vectors, comprising the aforementioned suitable DNA sequences and suitable promoters or control sequences. These vectors can be used to transform suitable host cells to enable them to express proteins. Vectors typically contain sequences for plasmid maintenance and for cloning and expression of exogenous nucleotide sequences. Such sequences (collectively referred to as "flanking sequences" in certain embodiments) typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting a nucleic acid encoding an antibody to be expressed, and a selectable marker element.

The host cells involved in the present invention can be prokaryotic cells, such as bacterial cells; or lower eukaryotic cells, such as yeast cells; or higher eukaryotic cells, such as mammalian cells. Representative examples include bacterial cells of *Escherichia coli, Streptomyces,* and *Salmonella typhimurium;* fungal cells of yeast; insect cells of Drosophila S2 or Sf9; animal cells such as CHO, COS7, 293 cells, etc.

In some embodiments of the present invention, the host cell can be various functional cells well known in the art, such as various killer cells, including but not limited to cytokine-induced killer (CIK) cells, dendritic cell-stimulated cytokine-induced killer (DC-CIK) cells, cytotoxic T lymphocytes (CTL), γδT cells, natural killer (NK) cells, tumor-infiltrating lymphocytes (TIL), lymphokine-activated killer (LAK) cells, CD3AK cells (anti-CD3 monoclonal antibody activated killer cells), and CAR-T/TCR-T cells. In some embodiments of the present invention, the killer cells are T cells or NK cells. Exemplary NK cells include, but are not limited to, primary NK cells, NK cell lines (such as NK92), and NKT cells. In some embodiments of the present invention, the NK cell is a primary NK cell. Exemplary T cells include, but are not limited to, peripheral blood T lymphocytes, umbilical cord blood T lymphocytes, cytotoxic T lymphocytes (CTLs), helper T cells, suppressor/regulatory T cells, γδ T cells, and T cells from mixed populations such as cytokine-induced killer cells (CIKs), and tumor-infiltrating lymphocytes (TILs). In some embodiments, the T cells are peripheral blood T lymphocytes or umbilical cord blood T lymphocytes.

Transformation of host cells with recombinant DNA can be performed using conventional techniques well known to those skilled in the art. When the host is a prokaryote such as *E. coli,* competent cells capable of taking up DNA can be harvested after the exponential growth phase and treated with $CaCl_2$, the steps of which are well known in the art. Another method is to use $MgCl_2$. Furthermore, transformation can also be performed using electroporation. When the host is a eukaryote, the following DNA transfection methods can be used: calcium phosphate co-precipitation method, conventional mechanical methods such as microinjection, electroporation, and liposome packaging.

The obtained transformants can be cultured using conventional methods to express the polypeptide encoded by the gene of the present invention. Depending on the host cells used, the culture medium used in the culture can be selected from various conventional media, such as serum-containing or serum-free media. Culture is carried out under conditions suitable for host cell growth. Once the host cells have grown to an appropriate cell density, the selected promoter is induced using a suitable method (such as temperature shift or chemical induction), and the cells are cultured for a further period.

The polypeptides in the above methods can be expressed intracellularly or on the cell membrane, or secreted extracellularly. If desired, the recombinant proteins can be isolated and purified by various separation methods based on their physical, chemical, and other properties. These methods are well known to those skilled in the art. Examples of such methods include, but are not limited to: conventional renaturation treatment, treatment with a protein precipitant (salting-out method), centrifugation, osmotic lysis, ultrafiltration, ultracentrifugation, molecular sieve chromatography (gel filtration), adsorption chromatography, ion-exchange chromatography, high-performance liquid chromatography (HPLC) and other various liquid chromatography techniques, and combinations of these methods.

The present invention further discloses vectors for cloning and expressing any one of the chimeric antigen receptors of the present invention. In some embodiments of the present invention, the vectors are adapted for replication and integration in eukaryotic cells, such as mammalian cells. In some embodiments of the present invention, the vectors are viral vectors. Examples of viral vectors include, but are not limited to, adenovirus vectors, adeno-associated virus vectors, retrovirus vectors, lentivirus vectors, vaccinia vectors, herpes simplex virus vectors, and derivatives thereof. Viral vector technology is well known in the art and has been described in Sambrook et al. (Molecular Cloning: A Laboratory Manual. Cold Spring Harbor Laboratory Press, Cold Spring Harbor. (2001)) and other virology and molecular biology manuals.

Numerous virus-based systems have been developed in the prior art for transferring genes into mammalian cells. For example, retroviruses provide a convenient platform for gene delivery systems. Heterologous nucleic acids can be inserted into vectors and packaged into retroviral particles using techniques known in the art. Recombinant viruses can then be isolated *in vitro* or *ex vivo* and delivered to engineered mammalian cells. Many retroviral systems are known in the art. In some embodiments of the present invention, adenoviral vectors are used. Many adenoviral vectors are known in the art. In some embodiments of the present invention, lentiviral vectors are used. In some embodiments of the present invention, self-inactivating lentiviral vectors are used. For example, self-inactivating lentiviral vectors carrying sequences encoding immunomodulators (e.g., immune checkpoint inhibitors) and/or self-inactivating lentiviral vectors carrying chimeric antigen receptors can be packaged using methods known in the art. Using methods known in the art, the resulting lentiviral vectors can be used to transduce mammalian cells (e.g., primary human T cells). Vectors derived from retroviruses (such as lentiviruses) are suitable tools for achieving long-term gene transfer because they allow for long-term, stable integration of transgenes and their proliferation in progeny cells. Lentiviral vectors also have the advantages of low immunogenicity and the ability to transduce non-proliferating cells.

In some embodiments of the present invention, the vector comprises any nucleic acid encoding the CAR in the present invention. The nucleic acid can be cloned into the vector using any molecular cloning method known in the art, including, for example, using restriction endonuclease sites and one or more selection markers. In some embodiments of the present invention, the nucleic acid is operatively linked to a promoter. A variety of promoters have been explored for gene expression in mammalian cells, and any promoter known in the art can be used in the present invention. Promoters can be further classified as constitutive promoters or regulated promoters, such as inducible promoters.

In some embodiments of the present invention, the nucleic acid encoding the CAR is operably linked to a constitutive promoter. Constitutive promoters allow constitutive expression of a heterologous gene (also referred to as a transgene) in a host cell. Exemplary constitutive promoters contemplated by the present invention include, but are not limited to, cytomegalovirus (CMV) promoter, human elongation factor-$1\alpha$ (hEF1$\alpha$) promoter, ubiquitin C (UbiC) promoter, phosphoglycerate kinase (PGK) promoter, simian virus 40 (SV40) early promoter, and chicken $\beta$-actin coupled with CMV early enhancer (CAGG) promoter. The efficiency of such constitutive promoters in driving transgene expression has been extensively compared in numerous studies. For example, Michael C. Milone et al. (Molecular Therapy, 17(8):1453-1464 (2009)) compared the efficiency of CMV, hEF1$\alpha$, UbiC, and PGK in driving chimeric antigen receptor expression in human primary T cells and concluded that the hEF1$\alpha$ promoter not only induced the highest level of transgene expression but also remains optimal in human CD4$^+$ and CD8$^+$ T cells. In some embodiments of the present invention, the nucleic acid encoding the CAR is operably linked to the hEF1$\alpha$ promoter.

In some embodiments of the present invention, the nucleic acid encoding the CAR is operably linked to an inducible promoter. Inducible promoters belong to regulated promoters. An inducible promoter can be induced by one or more conditions, such as physical conditions, microenvironment of engineered immune effector cells or physiological state of engineered immune effector cells, and inducing agents.

In some embodiments of the present invention, the inducing condition does not induce the expression of endogenous genes in the engineered mammalian cell and/or a subject receiving the pharmaceutical composition. In some embodiments of the present invention, the inducing condition is selected from the group consisting of: inducing agents, radiation (e.g., ionizing radiation, light), temperature (e.g., heat), redox states, tumor environments, and activation states of engineered mammalian cells.

In some embodiments of the present invention, the vector further comprises a selectable marker gene or a reporter gene for selecting CAR-expressing cells from a host cell population transfected by the lentiviral vector. Both selection markers and reporter genes may be flanked by appropriate regulatory sequences for expression in the host cell. For example, the vector may comprise transcription and translation terminators, initiation sequences, and promoters for regulating the expression of the nucleic acid sequence.

"Signal peptide": Signal peptide, sometimes also referred to as a signal sequence, targeting signal, localization signal, localization sequence, transport peptide, leader sequence or leader peptide, is a short peptide (typically 16-30 amino acids long) (Kapp, Katja; Schrempf, Sabrina; Lemberg, Marius K.; Dobberstein, Bernhard (2013-01-01)), located at the N-terminus of most newly synthesized proteins destined for a secretory pathway (occasionally located at the C-terminus or internally in non-canonical cases) (Owji, et al., A comprehensive review of signal peptides: Structure, roles, and applications, European Journal of Cell Biology. 97(6):422-441. (2018)) (Blobel G, Dobberstein B, et al., Transfer of proteins across membranes. I. Presence of proteolytically processed and unprocessed nascent immunoglobulin light chains on membrane-bound ribosomes of murine myeloma, The Journal of Cell Biology, 67(3):835-51 (1975)).

Signal peptides function to promote a cell to translate the protein, usually to the cellular membrane. In prokaryotes, signal peptides direct the newly synthesized protein to the SecYEG protein-conducting channel, which is present in the plasma membrane. A homologous system exists in eukaryotes, in which signal peptides direct the newly synthesized proteins to the Sec61 channel, which channel shares structural and sequence homology with SecYEG but is present in the endoplasmic reticulum (Rapoport TA, Protein translocation across the eukaryotic endoplasmic reticulum and bacterial plasma membranes, Nature. 450(7170):663-9 (2007)). The SecYEG and Sec61 channels are commonly referred to as translocons, and transport through these channels is known as translocation. As a secreted protein passes through the channel, transmembrane regions may diffuse through a lateral gate in the translocon to distribute into the surrounding membrane.

"Lentiviral vector": Lentiviral vector is a vector derived from a lentivirus, which comprises one or more lentiviral packaging proteins and lentiviral proteins necessary for the expression of one or more genes carried by the vector. It is produced by performing multiple attenuations on the virulence genes of lentiviruses such as HIV through technical means such as gene editing and genetic engineering. For example, deleting genes such as env, vif, vpr, vpu, and nef renders the lentiviral vector biologically safe.

Lentiviral vectors or retroviral vectors are generally packaged in packaging cells using lentiviral vector packaging systems or retroviral vector packaging systems. Illustratively, procedures and methods for packaging lentiviral vectors are described in Merten OW, et al., Production of lentiviral vectors. Mol Ther Methods Clin Dev. (2016), 3, 16017, which is incorporated herein by reference in its entirety.

Commonly used pseudotyped lentiviral vectors include the so-called third-generation lentiviral vector packaging system. The third-generation lentiviral vector packaging system includes four plasmids, generally a transfer vector and three packaging plasmids: a transfer vector encoding a gene of interest ("GOI"), e.g., a transgene, a GagPol plasmid, a Rev plasmid, and an envelope plasmid (containing a viral glycoprotein gene such as VSV-G or a variant thereof or Cocal-G or a variant thereof).

"Transfer vector" comprises a lentiviral vector backbone genome and a transgene. The transfer vector typically carries one or more transgenes flanked by long terminal repeat (LTR) sequences, which facilitates the integration of the transgenes contained in the transfer vector into the host genome. LTRs are responsible for the reverse transcription and integration of the viral genome. Through these sequences, lentiviruses can integrate transgenes into the genome of host cell. For safety reasons, transfer vectors are usually designed to render the resulting viral vector replication-incompetent. For example, transfer vectors lack the genetic elements necessary to produce infectious lentiviral particles in host cells. Furthermore, transfer vectors can be engineered to lack 3' LTR, thereby achieving "self-inactivating" of the virus. Compared with the conventional second-generation pseudotyped lentiviral vector packaging systems (usually a single packaging plasmid comprising nucleic acids encoding Gag, Pol, Rev, and Tat, and a separate envelope plasmid), the TAT gene can be eliminated from third-generation pseudotyped lentiviral vector packaging systems by incorporating a chimeric 5' LTR fused to a heterologous promoter (e.g., CMV or RSV promoter) on the transfer vector. Transfer vectors typically comprise a Ψ sequence (Psi sequence, also known as the Ψ packaging signal) downstream of the 5' LTR, wherein the Ψ sequence is responsible for packaging the transgenic RNA into the viral particle. The Ψ sequence ensures that only transgene-containing RNA is packaged into the viral particle. The transfer vector may also optionally comprise an Internal Ribosome Entry Site ("IRES") to allow simultaneous translation of two or more open reading frames (ORFs) on a single mRNA, thereby enabling multi-gene expression. Some transfer vectors, such as the lentiviral-GFP plasmid used in some embodiments of the present invention, may also contain selectable marker genes, such as antibiotic resistance genes (e.g., PuroR, encoding puromycin resistance) or fluorescent protein genes (e.g., GFP), for screening or tracking transduced cells.

For details regarding the transfer vector in the lentiviral vector packaging system, see Dull, et al., J. Virol. 72:8463-71 (1998); Miyoshi, et al., J. Virol. 72:8150-57 (1998) for details.

Exemplarily, the packaging plasmids include, but are not limited to, pMD2.G, pRSV-rev, pMDLG-pRRE, and pRRL-GOI.

Lentiviral vectors and lentiviral vector backbone genomes are known in the art (see Naldini, et al., (1996) Science 272:263-7; Zufferey, et al., (1998) J. Virol. 72:9873-9880; Dull, et al., (1998) J. Virol. 72:8463-8471; U.S. Patent No. 6,013,516; and U.S. Patent No. 5,994,136, each of which is incorporated herein by reference in its entirety.

"MOI": "Multiplicity of Infection (MOI)" refers to the number of viral particles added to each cell during the infection process. For example, when one million viral particles are added to one million cells, the MOI is 1.

As used in the present invention, "immune effector cells" are immune cells capable of performing immune effector functions. In some embodiments of the present invention, the immune effector cells express at least Fc$\gamma$RIII and perform ADCC effector functions. Examples of immune effector cells mediating ADCC include T cells, B cells, NK cells, macrophages, dendritic cells, and induced pluripotent stem cells (iPSCs).

In some embodiments of the present invention, the immune effector cells are T cells. In some embodiments of the present invention, the T cells are CD4$^+$/CD8$^-$, CD4$^-$/CD8$^+$, CD4$^+$/CD8$^+$, CD4$^-$/CD8$^-$ T cells, or combinations thereof. In some embodiments of the present invention, the T cells produce IL-2, TFN, and/or TNF after expressing CAR and binding to target cells such as CD79B$^+$ tumor cells. In some embodiments of the present invention, CD8$^+$ T cells lyse antigen-specific target cells upon expressing CAR and binding to target cells.

In some embodiments of the present invention, the immune effector cells are NK cells. In other embodiments, the immune effector cells may be established cell lines, such as NK-92 cells.

In some embodiments of the present invention, immune effector cells may be differentiated from stem cells, such as hematopoietic stem cells, pluripotent stem cells, iPSCs, or embryonic stem cells.

The engineered immune effector cells of the present invention are prepared by introducing the CAR into immune effector cells (e.g., T cells). In some embodiments of the present invention, the CAR is introduced into immune effector cells by transfecting any isolated nucleic acid encoding the CAR or any one of the above-mentioned vectors containing the nucleic acid encoding the CAR.

Methods for introducing vectors or isolated nucleic acids into mammalian cells are known in the art. The vectors described can be transferred to immune effector cells by physical, chemical, or biological methods.

Physical methods for introducing vectors into immune effector cells include calcium phosphate precipitation, lipid transfection, particle bombardment, microinjection, electroporation, etc. Methods for generating cells containing vectors and/or exogenous nucleic acids are well known in the art (see Sambrook, J., Fritsch, E.F. and Maniatis, T. (2001) Molecular Cloning: A Laboratory Manual. Cold Spring Harbor Laboratory Press, Cold Spring Harbor). In some embodiments of the present invention, vectors are introduced into cells via electroporation. In some embodiments of the present invention, vectors are introduced into cells via a PEI (Polyethylenimine) transfection reagent.

Biological methods for introducing vectors into immune effector cells include the use of DNA and RNA vectors. Viral vectors have become the most widely used method for inserting genes into mammalian cells (such as human cells).

Chemical methods for introducing vectors into immune effector cells include colloidal dispersion systems, such as macromolecular complexes, nanocapsules, microspheres, beads, and lipid-based systems, such as oil-in-water emulsions, micelles, mixed micelles, and liposomes. An exemplary colloidal system useful as a delivery vector *in vitro* is a liposome.

In some embodiments of the present invention, RNA molecules encoding any one of the CARs described herein can be prepared by conventional methods (e.g., *in vitro* transcription) and then introduced into immune effector cells by known methods such as mRNA electroporation (see Peter M Rabinovich. Human Gene Therapy, 17:1027-1035 (2006)).

In some embodiments of the present invention, upon introduction of the vector or isolated nucleic acid, the transduced or transfected immune effector cells proliferate *ex vivo* or proliferate *in vivo* in a subject administered with the transfected/transduced immune effector cells. In some embodiments of the present invention, the transduced or transfected immune effector cells are cultured to proliferate for at least about 1, 2, 3, 4, 5, 6, 7, 8, 9, 10, 11, 12, 13, or 14 days. In some embodiments of the present invention, the transduced or transfected immune effector cells may be further evaluated or screened to select engineered immune effector cells.

Reporter genes can be used for identifying potentially transfected cells and evaluating functionality of regulatory sequences. Generally, a reporter gene is a gene that is not present in or expressed by the recipient organism or tissue, and encodes a polypeptide that expresses some readily detectable properties, such as enzymatic activity. Expression of the reporter gene is determined at appropriate time after introduction of the DNA into the recipient cell. Suitable reporter genes may include genes encoding luciferase, $\beta$-galactosidase, chloramphenicol acetyltransferase, secreted alkaline phosphatase, or green fluorescent protein (see Kumiko Ui-Tei. FEBS Letters, 479:79-82 (2000)). Suitable expression systems are known in the art and can be prepared using known techniques or obtained commercially.

Other methods for confirming the presence of a nucleic acid encoding a CAR in an engineered immune effector cell include: molecular biological assays well known to those skilled in the art, such as Southern and Northern blotting, RT-PCR, and PCR; biochemical assays, such as detecting the presence or absence of a specific peptide; and immunological methods, such as ELISA.

The compositions disclosed in the present invention comprise a pharmaceutically acceptable excipient or carrier and any one of the following components: any one of the aforementioned anti-CD79B single-domain antibodies, CARs, and engineered immune effector cells disclosed in the present invention. Pharmaceutically acceptable excipients or carriers include, but are not limited to, diluents, solubilizers, emulsifiers, preservatives, and/or adjuvants. The auxiliary substances are preferably non-toxic or substantially non-toxic to recipients at the dose and concentration used. Such auxiliary substances include, but are not limited to, saline, buffers, glucose, water, glycerol, ethanol, and combinations thereof. In some embodiments, the pharmaceutical composition may contain substances for improving, maintaining, or retaining, for example, pH, osmolality, viscosity, clarity, color, isotonicity, odor, sterility, stability, dissolution or release rate, absorption, or permeability of the composition. The optimal pharmaceutical composition can be determined based on the intended route of administration, delivery mode, and required dosage.

Pharmaceutical compositions for *in vivo* administration are typically provided as sterile formulations. Sterilization is achieved by filtration through a sterile filter membrane. If the composition is lyophilized, sterilization using this method can be performed before or after lyophilization, reconstitution, or dilution. The pharmaceutical compositions of the present invention may be used for parenteral delivery. Compositions for parenteral administration can be stored in lyophilized form or in solution. They are prepared, for example, by conventional methods using physiological saline or aqueous solutions containing glucose and other auxiliary agent. Parenteral compositions are typically placed in containers with a sterile access port, such as intravenous solution bags or vials with a stopper penetrable by a hypodermic injection needle. Or, the compositions may be used for inhalation or delivery via the digestive tract (e.g., orally). The preparation of the pharmaceutically acceptable compositions is within the technical scope of the art. Other pharmaceutical compositions would be apparent to those skilled in the art, including formulations containing antibodies in sustained- or controlled-release delivery formulations. Techniques for formulating a variety of other sustained or controlled delivery modes (such as liposome carriers, bioerodible microparticles or porous beads, and depot injections) are also known to those skilled in the art.

Once formulated, the pharmaceutical composition is stored in sterile vials in the form of a solution, suspension, gel, emulsion, solid, crystal, or lyophilized powder. The formulation can be stored in a ready-to-use form or in a form for reconstitution prior to administration (e.g., lyophilized form). The present invention further provides kits for generating single-dose administration units. The kits of the present invention may each contain a first container with dried protein and a second container with an aqueous formulation. In some embodiments of the present invention, the kits comprising single-chamber and multi-chamber pre-filled syringes (e.g., liquid syringes and syringes for lyophilized formulation) are provided.

The present invention further provides a method for treating a patient (especially a patient with a CD79B-related disease) by administering the anti-CD79B single-domain antibody, engineered immune effector cell, or pharmaceutical composition thereof according to any embodiment of the present invention. The terms "patient," "subject," "individual," and "object" are used interchangeably in the present invention and include any living organism, preferably an animal, more preferably a mammal (e.g., rat, mouse, dog, cat, and rabbit), and most preferably a human.

"Treatment" refers to administration to a subject of a therapeutic method described herein to achieve at least one positive therapeutic effect (e.g., reduced number of cancer cells, decreased tumor volume, reduced rate of cancer cell infiltration into surrounding organs, or reduced rate of tumor metastasis or tumor growth). Therapeutic methods effective to treat a patient may vary depending on multiple factors (such as the patient's disease state, age, body weight, and ability of the therapy to elicit an anti-cancer response in the subject).

"Therapeutically effective amount": As used herein, a "therapeutically effective amount" is the amount of a composition or an active substance thereof that is sufficient to confer a beneficial effect on an individual to whom it is administered, or otherwise reduces adverse, non-beneficial events, such as the amount of the anti-CD79B single-domain antibody and CAR-T cells provided in the present invention. Exemplarily, a "therapeutically effective dose" herein refers to a dose that produces one or more desired or expected (e.g., beneficial) effects as a result of its administration, which is carried out once or multiple times over a specified period. The exact dose will depend on the purpose of the treatment and can be determined by those skilled in the art using known techniques (see, e.g., Lieberman, Pharmaceutical Dosage Forms (Vols. 1-3, 1992); Lloyd, The Art, Science and Technology of Pharmaceutical Compounding (1999); and Pickar, Dosage Calculations (1999)).

A therapeutically effective amount of a pharmaceutical composition comprising the anti-CD79B single-domain antibody or engineered immune effector cell, such as CAR-T cell, disclosed in the present invention to be employed will depend on, for example, the degree and the purpose of treatment. Those skilled in the art would understand that the appropriate dose level for treatment will vary in part depending on the molecule delivered, indication, route of administration, and condition (body weight, body surface, or organ size) and/or status (age and general health) of the patient. In some embodiments, clinicians may titrate the dose and change the route of administration to obtain optimal therapeutic effect.

The frequency of administration will depend on the pharmacokinetic parameters of the anti-CD79B single-domain antibodies or engineered immune effector cells in the formulation used. Clinicians typically administer the pharmaceutical composition until a dosage that achieves the desired effect is reached. The pharmaceutical composition can thus be administered as a single dose, or over time as two or more doses (which may or may not contain the same amount of desired molecules), or as a continuous infusion through an implantable device or catheter.

The routes of administration/delivery of pharmaceutical compositions are conventional in the art, such as oral, nasal, intravenous, intraperitoneal, intracerebral (intraparenchymal), intracerebroventricular, intramuscular, intraocular, intraarterial, portal venous, or intralesional injection routes, and administration can also be carried out via sustained-release systems or implantable devices.

"And/or": It should be understood to mean one or two alternative options.

"About" / "approximately": As used herein, the term "about" or "approximately" refers to a quantity, level, value, number, frequency, percentage, dimension, size, amount, weight, or length that varies by up to 15%, 10%, 9%, 8%, 7%, 6%, 5%, 4%, 3%, 2%, or 1% compared with a reference quantity, level, value, number, frequency, percentage, dimension, size, amount, weight, or length. In an embodiment, the term "about" or "approximately" refers to a range of quantity, level, value, number, frequency, percentage, dimension, size, amount, weight, or length varying by $\pm15\%$, $\pm10\%$, $\pm9\%$, $\pm8\%$, $\pm7\%$, $\pm6\%$, $\pm5\%$, $\pm4\%$, $\pm3\%$, $\pm2\%$, or $\pm1\%$ relative to a reference quantity, level, value, number, frequency, percentage, dimension, size, amount, weight, or length. It is understood that reference herein to "about" a value, range, or parameter includes (and describes) embodiments directed to the value, range, or parameter itself. For example, reference to "about X" includes description of "X."

"Comprising": As used herein, unless the context otherwise requires, the term "comprising" will be construed to mean including specified step, element, or group of steps or elements, but not excluding any other steps, elements, or group of steps or elements. In some embodiments of the present invention, the terms "including," "having," "containing" and "comprising" are used synonymously.

"Embodiment": Throughout this specification, references to "some embodiments," "embodiments," "certain embodiments," or combinations thereof mean that a particular feature, structure, or characteristic described in connection with the embodiments is included in at least one embodiment of the present invention. Therefore, the appearances of these phrases in various places throughout this specification do not necessarily refer to the same embodiment. Furthermore, the particular features, structures, or characteristics can be combined in any suitable manner in one or more embodiments.

"Operably": A nucleic acid is "operably linked" when it is placed into a functional relationship with another nucleic acid. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the nucleic acid sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

All publications, documents, and patents mentioned herein are hereby incorporated by reference in their entirety as if each individual publication, document, or patent not specifically and individually indicated to be incorporated by reference in its entirety were so incorporated. In the event of a conflict, the present invention (including any definitions herein) shall prevail. However, any reference, article, publication, patent, patent publication, and patent application cited herein is not and should not be taken as an admission or any kind of suggestion.

The section headings used herein are for organizational purposes only and are not to be construed as limiting the subject matter described.

## BRIEF DESCRIPTION OF THE DRAWINGS

FIG. 1: FIG. 1 is a flow cytometry diagram showing specific binding of the anti-CD79B single-domain antibodies JYB4, JYB10, JYB8, JYB11, and JYB14 to the CD79B antigen; and

FIG. 2: FIG. 2 is a graph showing killing efficiency of the CAR-T-JYB4 cells, CAR-T-JYB10 cells, CAR-T-JYB8 cells, CAR-T-JYB11 cells, and CAR-T-JYB14 cells against target Ramos cells.

## DETAILED DESCRIPTION OF THE EMBODIMENTS

The concept of the present invention and technical effects produced will be clearly and completely described below with reference to examples, so as to fully understand the objective, features, and effects of the present invention. Obviously, only some but not all examples of the present invention are described. Other examples obtained by those skilled in the art based on the examples of the present invention without inventive efforts are all within the scope of protection of the present invention.

In the following examples, experimental methods for which specific conditions are not indicated are performed according to conventional methods and conditions known in the art, or according to the manufacturer's instructions. Reagents and raw materials for which specific components are not specified in the present invention are all commercially available.

Example 1

Immunization of animals and establishment of a phage library to prepare anti-CD79B single-domain antibodies.

A. Animal Immunization and Immune Response Assay

(1) Healthy alpacas were selected as immunization subjects.

(2) For the first immunization, complete Freund's adjuvant was mixed with CD79B antigen (0.4 mg) at a ratio of 1:1, emulsified, and then administered by multi-point subcutaneous injection. For subsequent booster immunizations, incomplete Freund's adjuvant was mixed with CD79B antigen at a ratio of 1:1. Immunizations were performed at 2-week intervals for a total of 4 immunizations. 5 mL of peripheral blood was collected prior to the immunization and after each immunization. Serum was isolated and immune responses were monitored by ELISA to confirm serum titers.

CD79B antigen used for animal immunization:
Trade name: Human CD79A&CD79B Heterodimer Protein, Mouse IgG2a Fc, Flag Tag&Mouse IgG2a Fc, His Tag (MALS verified); Cat. No.: #CDB-H52W3-1mg; Brand: ACRO.
Antigen used for ELISA detection: Human CD79B Protein, His Tag (MALS verified), Cat. No.: #CDB-H52H3-1mg; Brand: ACRO.
(3) After the fourth immunization, the plasma titer reached a level of 10,000. 20 mL of blood was collected, and lymphocytes were isolated therefrom and stored in TRIzol reagent for subsequent construction of the antibody phage library.

B. Construction of Antibody Phage Library

(1) Reverse Transcription
After completion of the animal immunization, total RNA was extracted from the isolated peripheral blood lymphocytes and reverse transcription was performed on the total RNA using the Takara reverse transcription kit. The total RNA sample was divided into two aliquots: one aliquot was reverse-transcribed into cDNA using Oligo dT Primer provided in the kit as a primer, and the other aliquot was reverse-transcribed into cDNA using Random 6-mers provided in the kit as a primer, according to the instructions of the reverse transcription kit. The resulting products were stored separately in two centrifuge tubes.
(2) PCR Amplification

a. PCR amplification was performed to amplify specific antibody fragments from the reverse-transcribed cDNA using Taq DNA Polymerase Hot Start enzyme. All PCR products were subjected to 1% agarose gel electrophoresis, and bands with a target fragment size of about 600-700 bp were excised and recovered as the first-round PCR amplification products, which were stored at -20 °C.
b. The first-round PCR amplification products were used as a template for the second-round PCR reaction. Upon completion of the reaction, 1% agarose gel electrophoresis was performed. Finally, a single target band with a fragment size of about 400 bp was excised and recovered, and the PCR reaction solution was then subjected to DNA purification using a universal DNA purification kit.

(3) Enzyme Digestion and Ligation
The target gene fragment amplified by the second-round PCR and the pComb3XSS phage plasmid vector were digested with restriction endonucleases SpeI and SacI, respectively. After digestion, the VHH target gene fragment was ligated into the pComb3XSS phage plasmid vector using a ligase to construct a recombinant plasmid.
(4) Construction of Bacterial Library

a. 50 μL of TG1 competent cells were placed on ice for 5-10 min to thaw.
b. 100 ng of the ligation product was added, transferred into pre-chilled electroporation cuvettes with a spacing of 1 mm. The electroporator was set with the following parameters: 1800 V, 1 mm, and transformation was performed by pressing.
c. Immediately after completion of electroporation, 1 mL of SOC medium pre-warmed at 37 °C was added. After mixing thoroughly, the cells were recovered by shaking at 37 °C and 200 rpm for 1 h.

d. Twenty-one 100 ng ligation systems were used for performing the electroporation with competent cells according to the above method.

e. 100 µL of the recovered bacterial culture was taken and subjected to 10-fold serial dilutions, then plated, and incubated at 37 °C overnight.

f. All remaining bacterial culture was collected and evenly spread onto five 245 mm square culture plates (2×YT containing 100 µg/mL Amp, 2% agarose), and incubated inverted at 37 °C overnight.

g. The number of transformant colonies obtainable from all reactions was calculated according to the dilution factor and the number of single colonies, which was the library capacity of the bacterial library. Meanwhile, several single clones were randomly selected from the gradient dilution plate for colony PCR. Clones showing a single band of approximately 400 bp in the PCR products were considered positive clones, based on which the cloning positive rate of the bacterial library was estimated.

h. Colonies from the overnight cultured plates were scraped off using 2×YT liquid medium, transferred into a 50 mL centrifuge tube, and measured for their OD600 value. Glycerol was added to a final concentration of 20% for storage at -80 °C.

(5) Construction of Phage Library

a. The bacterial library was inoculated into 100 mL of 2×YT liquid medium (containing 100 µg/mL Amp) to an initial OD600 value of 0.1, and incubated at 37 °C and 250 rpm until the OD600 reached 0.5-0.55.

b. Helper phage was added at a ratio of 1:20 (the number of bacteria : the number of phage), followed by incubation at 37 °C and 250 rpm for 30 min.

c. Kana (Kanamycin) was added to a final concentration of 50 µg/mL. The mixture was incubated at 30 °C and 250 rpm overnight, followed by centrifugation to collect the supernatant.

d. 1/4 volume of pre-chilled PEG/2.5 M NaCl was added, mixed thoroughly, left to stand on ice and incubated for at least 30 min. After centrifugation at 4 °C and 4000 rpm for 20 min and removal of the supernatant, the pellet was dissolved in 1 mL of PBS buffer. 1/4 volume of pre-chilled PEG/NaCl was added again, followed by incubation on ice for 10 min. After centrifugation at 4 °C and 12000 g for 10 min and removal of the supernatant, the pellet was dissolved in 1 mL of PBS and stored at -80 °C to obtain the purified phage library.

C. <u>Phage Screening</u>

(1) First Round of Screening

a. The screening antigen (the above Human CD79B Protein, His Tag) was retrieved from the -80 °C freezer and thawed on ice.

b. Immunotubes were coated with the screening antigen (40 µg/tube, coating buffer: CBS, pH 9.6, 2 mL/tube), and rotated slowly at 4 °C overnight. Another immunotube was coated with BSA in parallel as a control.

c. The liquid in the overnight coated immunotubes was discarded, and 2 mL of PBS buffer was added to wash the immunotubes 3 times at room temperature, with rotation for 5 min each time.

d. 2 mL of blocking solution (3% BSA) was added, followed by blocking with rotation at room temperature for 2 h.

e. The liquid in the blocked immunotubes was discarded, and 2 mL of PBS buffer was added to wash the immunotubes 3 times at room temperature, with rotation for 5 min each time.

f. The washing solution in the immunotubes was discarded, and 2 mL of PBS was added. The prepared phage library was calculated according to the following formula and added. 28 µL (approximately $10^{12}$ pfu) was used as the phage library input for the first round of screening, and incubated with rotation at room temperature for 1 h.

$$V(ul) = \frac{10^{12}}{T_{library}} \times 1000$$

where V is the volume of phage added (unit: µL), and $T_{library}$ is the phage titer.

g. The liquid in the immunotubes was discarded, and 2 mL of PBST buffer (1× PBS plus 0.1% Tween 20, the same applies hereinafter) was added to wash the immunotubes 20 times at room temperature, with rotation for 5 min each time.

h. The liquid in the immunotubes was discarded, and residual liquid was removed as thoroughly as possible. Then 1 mL of 0.25 mg/mL Trypsin solution was added, and elution was carried out with rotation at room temperature for 30 min.

i. 10 μL of 10% AEBSF was added to terminate the elution, and the solution in the immunotubes was transferred into a new 1.5 mL centrifuge tube to obtain the first-round screening phage eluate.

(2) Determination of Titer of the First-Round Phage Eluate

a. The TG1 strain stored in the -80 °C freezer was streaked on 2×YT solid medium for single colonies, followed by overnight culture at 37 °C (stored at 4 °C for one week). A single colony was picked from the single-colony plate into 5 mL of 2×YT medium, and incubated at 37 °C overnight.

b. 500 μL of the overnight bacterial culture was transferred into 5 mL of 2×YT liquid medium and incubated at 37 °C and 250 rpm for approximately 45-60 min until the OD600 reached 0.5-0.55.

c. 10 μL of the first-round phage eluate was subjected to 10-fold serial dilutions in 1.5 mL centrifuge tubes for a total of 12 dilutions. Specifically, 10 μL of the first-round phage eluate was diluted to 100 μL, and then 10 μL of the resulting dilution was further diluted to 100 μL, and so on, until a $10^{-12}$ dilution was obtained after a total of 12 dilutions, followed by vortexing to mix thoroughly.

d. 90 μL of TG1 bacterial culture was added to each dilution centrifuge tube, mixed thoroughly, and incubated at 37 °C for 30 min.

e. 5 μL from each dilution tube was added dropwise onto 2×YT solid medium (Amp) and incubated inverted at 37 °C overnight.

f. The number of single colonies at a dilution that allowed clear distinction of individual colonies on the plate was counted, and the number of phagemids per milliliter of phage solution, i.e., the titer of the phage library, was calculated according to the following formula:

$$T \ (pfu/ml) \ = N \ \times D \ \times 400$$

wherein T is the phage titer (unit: pfu/mL), D is the dilution factor, and N is the number of single colonies at the corresponding dilution factor.

(3) Amplification of the First-Round Phage Eluate

a. The TG1 strain stored in the -80 °C freezer was streaked in advance on 2×YT solid medium for single colonies, followed by overnight culture at 37 °C (stored at 4 °C for one week). A single colony was picked from the single-colony plate into 5 mL of 2×YT medium, and incubated at 37 °C overnight.

b. 500 μL of the overnight bacterial culture was transferred into 5 mL of 2×YT liquid medium and incubated at 37 °C and 250 rpm for approximately 45-60 min until the OD600 value reached 0.5-0.55.

c. 500 μL of the phage eluate obtained from the first-round screening was added to the bacterial culture with the OD600 of 0.5-0.55 (the remaining eluate was stored at 4 °C).

d. Incubation was continued at 37 °C and 250 rpm for 30 min.

e. All the bacterial culture was evenly spread onto a 245 mm square culture plate containing 2% agarose and 100 μg/mL Amp, and incubated at 37 °C overnight.

f. 6 mL of 2×YT liquid medium (containing 100 μg/mL Amp) was added onto the surface of the overnight cultured square plate. Colonies on the square plate were gently scraped off using a spreader, and the bacterial culture was collected into a 15 mL centrifuge tube to obtain the amplified bacterial sub-library. The OD600 value of the bacterial culture was measured using a spectrophotometer as the OD600 value of the eluate bacterial library. Glycerol was added to a final concentration of 20% to obtain the first-round bacterial library.

g. A corresponding volume of the bacterial culture for the eluate bacterial library was calculated according to the following formula and transferred into 100 mL of 2×YT liquid medium (containing 100 μg/mL Amp) to achieve an initial OD600 of 0.1:

$$V \ (ul) = \frac{10}{OD600} \times 1000$$

wherein V is the volume of the transferred bacterial culture (unit: μL), and OD600 is the OD600 of the constructed eluate bacterial library.

h. Incubation was carried out at 37 °C and 250 rpm until the OD600 of the bacterial culture reached 0.5-0.55.

i. Helper phage M13K07 was calculated according to the following formula and added to achieve a ratio of the number of bacteria : the number of phage = 1:20:

$$V \ (ml) \ = \frac{OD600 \ \times 1.6 \ \times 10^{12}}{T_{helper-phage}}$$

wherein V is the volume of the helper phage added (unit: mL), $T_{helper-phage}$ is the titer of the helper phage used, and OD600 is the OD600 value of the bacterial culture.

j. Incubation was continued at 37 °C and 250 rpm for 30 min.

k. Kana was added to a final concentration of 50 μg/mL, and incubation was carried out at 30 °C and 250 rpm overnight.

(4) Purification of the First-Round Phage

a. The overnight bacterial culture was transferred into a new 50 mL centrifuge tube and centrifuged at 4 °C and 4000 rpm for 10 min.

b. The supernatant after centrifugation was transferred into a new 50 mL centrifuge tube, and 1/4 volume of pre-chilled 20% PEG/2.5 M NaCl at 4 °C was added. After thorough mixing, the mixture was placed on ice for 30 min.

c. Centrifugation was performed at 4 °C and 4000 rpm for 20 min. The supernatant was discarded, and the tube was inverted on paper for 2 min.

d. 1 mL of PBS was added to resuspend the pellet. The resuspended solution was transferred into a new 1.5 mL centrifuge tube and centrifuged at 4 °C and 12000 rpm for 20 min.

e. The supernatant after centrifugation was transferred into a new 1.5 mL centrifuge tube, and 1/4 volume of pre-chilled 20% PEG/2.5 M NaCl solution was added. After thorough mixing, the mixture was placed on ice for 10 min.

f. Centrifugation was performed at 4 °C and 13000 rpm for 10 min. The supernatant was discarded, and 1 mL of PBS was added to resuspend the pellet.

g. Centrifugation was performed at 4 °C and 13000 rpm for 2 min. The supernatant was transferred into a new 1.5 mL centrifuge tube to obtain the first-round screening phage sub-library, which was stored at -80 °C for long-term storage or at -20 °C for short-term storage (1-2 weeks).

h. The titer of the first-round screening phage sub-library was determined using the same method as described in C(2) in the foregoing.

(5) Second Round of Screening

Screening was performed using the same method as described in C(1) in the foregoing. 0.25 mL of the phage sub-library obtained from the first-round screening was used as the phage library input for the second round of screening to obtain the second-round screening phage eluate.

(6) Determination of Titer of the Second-Round Phage Eluate

The method was the same as that described in C(2) in the foregoing.

(7) Monoclonal ELISA Assay (Phage-ELISA)

After immunotube screening, 192 single clones were randomly selected for ELISA assay and secondary validation of positive clones.

a. The TG1 strain stored in the -80 °C freezer was streaked in advance on 2×YT solid medium for single colonies, followed by overnight culture at 37 °C (stored at 4 °C for one week). A single colony was picked from the single-colony plate into 5 mL of 2×YT medium, and incubated at 37 °C overnight.

b. 500 μL of the overnight bacterial culture was transferred into 5 mL of 2×YT liquid medium and incubated at 37 °C and 250 rpm for approximately 45-60 min until the OD600 value reached 0.5-0.55.

c. 10 μL of the phage eluate obtained from the second-round screening was subjected to 10-fold serial dilutions in 1.5 mL centrifuge tubes for a total of 12 dilutions. Specifically, 10 μL of the phage library was diluted to 100 μL, and then 10 μL of the resulting dilution was further diluted to 100 μL, and so on, for a total of 12 dilutions, followed by vortexing to mix thoroughly.

d. 90 μL of the bacterial culture with the OD600 value of 0.5-0.55 was added to each dilution centrifuge tube and mixed uniformly.

e. Incubation was continued at 37 °C and 250 rpm for 30 min.

f. The bacterial culture was spread evenly onto a solid medium plate containing 100 μg/mL Amp and incubated at 37 °C overnight.

g. Single clones were randomly picked from the overnight and cultured plates into sterile a 96-well cell culture plate (P1-P3). 200 μL of 2×YT medium (containing 100 μg/mL Amp) was added to each well, followed by static incubation at 37 °C overnight.

h. 2 μL of the overnight bacterial culture was transferred into a new 96-well cell culture plate containing 200 μL of

2×YT liquid medium (containing 100 $\mu$g/mL Amp) per well, followed by static incubation at 37 °C for 3-5 h. The overnight bacterial culture before transfer was stored at 4 °C.

i. Helper phage M13K07 was calculated according to the following formula and added to each well to achieve a ratio of the number of bacteria : the number of phage = 1:20:

$$V\ (ml) = \frac{OD600 \times 1.6 \times 10^{12}}{T_{helper-phage}}$$

wherein V is the volume of the helper phage added (unit: mL), and $T_{helper-phage}$ is the titer of the helper phage used.

j. Incubation was carried out at 37 °C for 30 min. Kana was added to a final concentration of 50 $\mu$g/mL, followed by static incubation at 30 °C overnight.

k. The overnight cultured 96-well plate was centrifuged at 4 °C and 4000 rpm for 10 min and stored at 4 °C for later use.

l. Microplates were coated with the screening antigen (1 ng/$\mu$L, coating buffer: CBS, pH 9.6, 100 $\mu$L/well). Another microplate was coated with BSA in parallel as a control. Coating was performed at 4 °C overnight.

m. The liquid in the overnight coated microplates was discarded. 200 $\mu$L of PBS buffer was added to each well, and the microplates were washed at room temperature 3 times, 10 min each time.

n. 200 $\mu$L of blocking solution (3% BSA) was added to each well, and the microplates were blocked at room temperature for 1 h.

o. The blocking solution was discarded. 200 $\mu$L of PBST buffer (1× PBS plus 0.1% Tween 20, the same applies hereinafter) was added to each well, and the microplates were washed at room temperature 3 times, 10 min each time.

p. 120 $\mu$L of 3% BSA was added to each well, followed by addition of 80 $\mu$L of the supernatant after centrifugation in step (7)(k), and incubation at room temperature for 2 h.

q. The liquid in the microplates was discarded. 200 $\mu$L of PBST buffer was added to each well for washing 3 times, 10 min each time.

r. M13 Bacteriophage Antibody (HRP), Mouse Mab, diluted 1:55000 in PBS, was added to each well at 100 $\mu$L/well, followed by incubation at room temperature for 1 h.

s. The liquid in the ELISA plate was discarded. 200 $\mu$L of PBST buffer was added to each well for washing 3 times, 10 min each time.

t. 100 $\mu$L of TMB single-component chromogenic solution was added to each well, and color development was performed in the dark for 2-3 min. 100 $\mu$L of 1 M HCl was added to each well to terminate the reaction. The OD450 nm value was read using a microplate reader, recorded, and saved.

(8) Secondary ELISA Validation of Positive Clones

To exclude false-positive results, secondary ELISA validation was performed using the same method as described in C(7) above.

(9) Sequencing of Positive Clones

Positive single clones were selected according to the ELISA detection data and secondary validation data. 5 $\mu$L of the positive clone bacterial culture taken from the plate in (g) in (7) monoclonal ELISA assay was inoculated into 1 mL of 2×YT medium (containing 100 $\mu$g/mL Amp), followed by incubation at 37 °C and 250 rpm until the OD600 reached 0.8-1.0 (approximately 6-8 h). 0.5 mL of the bacterial culture was sequenced, and glycerol was added to the remaining bacterial culture to a final concentration of 20%, followed by storage at -20 °C.

(10) Results of Two Rounds of Screening

The results of the two rounds of screening are shown in Table 2 below.

Table 2

| Screening Round | Input Titer (pfu) | Screening Titer (pfu/mL) | Control Titer (pfu/mL) | Enrichment Factor | Fold Difference |
|---|---|---|---|---|---|
| Round 1 | $1.0 \times 10^{12}$ | $1.0 \times 10^{8}$ | $1.0 \times 10^{8}$ | $1.0 \times 10^{-4}$ | 1 |
| Round 2 | $8.0 \times 10^{12}$ | $4.4 \times 10^{9}$ | $4.8 \times 10^{8}$ | $5.5 \times 10^{-4}$ | 9 |

D. ELISA Assay for Binding of Purified Single-Domain/VHH Antibodies to Various Antigens

Multiple validated positive monoclonal clones were subjected to gene sequencing to obtain multiple different anti-CD79B single-domain antibody sequences. After preparation and purification of multiple antibodies, they were respectively subjected to ELISA assay against human CD79B antigen, mouse CD79B antigen, human CD79A+CD79B antigen, and mouse CD79A+CD79B antigen, thereby screening out anti-CD79B single-domain antibodies JYB4, JYB10, JYB8, JYB11, and JYB14 capable of specifically binding to human CD79B antigen. The specific detection method is as follows.

(1) Microplates were coated with antigen (1 ng/$\mu$L, coating buffer: CBS, pH 9.6, 100 $\mu$L/well). Another microplate was coated with BSA in parallel as a control. Coating was performed at 4 °C overnight.
(2) The liquid in the overnight coated microplates was discarded. 200 $\mu$L of PBS buffer was added to each well, and the microplates were washed at room temperature 3 times, 10 min each time.
(3) 200 $\mu$L of blocking solution (3% BSA) was added to each well, and the microplates were blocked at room temperature for 1 h.
(4) The blocking solution was discarded. 200 $\mu$L of PBST buffer (1$\times$ PBS plus 0.1% Tween 20, the same applies hereinafter) was added to each well, and the microplates were washed at room temperature 3 times, 10 min each time.
(5) The purified nanobody/single-domain antibody was diluted to 0.1 $\mu$g/$\mu$L with PBS, and 500 $\mu$L was subjected to 10-fold serial dilutions for a total of 6 dilutions. Specifically, 50 $\mu$g of nanobody was diluted to 500 $\mu$L, and then 50 $\mu$L of this dilution was taken and diluted to 500 $\mu$L, and so on, for a total of 6 dilutions.
(6) 200 $\mu$L of nanobody at each dilution was added to each well, and incubated at room temperature for 2 h.
(7) The liquid in the microplates was discarded. 200 $\mu$L of PBST buffer was added to each well for washing 3 times, 10 min each time.
(8) Anti-myc antibody diluted 1:10000 in PBS was added to each well at 100 $\mu$L/well, followed by incubation at room temperature for 1 h.
(9) The liquid in the microplates was discarded. 200 $\mu$L of PBST buffer was added to each well for washing 6 times, 5 min each time.
(10) 100 $\mu$L of TMB single-component chromogenic solution was added to each well, and color development was performed in the dark for 2-3 min. 100 $\mu$L of 1 M HCl was added to each well to terminate the reaction. The OD450 nm value was read using a microplate reader, recorded, and saved.

The detection results are respectively shown in Tables 3-7 below.

Table 3

| JYB4 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ug | 0.5516 | 0. 0562 | 0. 6577 | 0.0490 | 0. 0488 |
| 2ug | 0.4848 | 0.0526 | 0.5846 | 0.0549 | 0. 0479 |
| 200ng | 0.3753 | 0.0525 | 0.4215 | 0.0567 | 0. 049 |
| 20ng | 0.1504 | 0.0519 | 0. 1723 | 0.0572 | 0.0499 |
| 2ng | 0.0519 | 0.0530 | 0.0630 | 0.0574 | 0.0479 |
| 200pg | 0.0521 | 0.0530 | 0.0577 | 0.0601 | 0.0476 |

Table 4

| JYB10 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ug | 0.2846 | 0.0982 | 0.8161 | 0. 1263 | 0. 0493 |
| 2ug | 0.2206 | 0.0588 | 0.5544 | 0.0788 | 0.0482 |
| 200ng | 0. 1437 | 0.0527 | 0.2237 | 0.0601 | 0.0473 |

(continued)

| JYB10 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ng | 0.0739 | 0.0517 | 0.0687 | 0.0609 | 0.0474 |
| 2ng | 0.0517 | 0.0512 | 0.0596 | 0.0584 | 0. 0486 |
| 200pg | 0. 0496 | 0.0517 | 0.0573 | 0.0584 | 0. 0488 |

Table 5

| JYB8 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ug | 0. 5958 | 0.2292 | 0.9220 | 0.3560 | 0.0537 |
| 2ug | 0. 5064 | 0.1779 | 0.8022 | 0.2065 | 0.0582 |
| 200ng | 0.3079 | 0.0622 | 1.4300 | 0.0680 | 0.0520 |
| | | | | | |
| 20ng | 0. 1446 | 0.0551 | 0.1149 | 0.0553 | 0.0554 |
| 2ng | 0.0600 | 0.0512 | 0.0544 | 0.0543 | 0.0474 |
| 200pg | 0.0418 | 0.0485 | 0.0521 | 0.0619 | 0.0534 |

Table 6

| JYB11 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ug | 0.3794 | 0.0679 | 0.6119 | 0.0981 | 0.0489 |
| 2ug | 0.3035 | 0.0541 | 0.5194 | 0. 0651 | 0.0483 |
| 200ng | 0. 1768 | 0.0515 | 0.2710 | 0.0589 | 0.0480 |
| 20ng | 0. 0947 | 0.0513 | 0.0778 | 0.0575 | 0.0471 |
| 2ng | 0.0528 | 0.0174 | 0.0567 | 0.0582 | 0.0485 |
| 200μg | 0.0516 | 0.0496 | 0.0556 | 0.0586 | 0.0469 |

Table 7

| JYB14 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 20ug | 0.4948 | 0. 0859 | 0.8551 | 0.1172 | 0. 0489 |
| 2ug | 0.3460 | 0.0602 | 0.6336 | 0.0726 | 0. 0506 |
| 200ng | 0.3212 | 0.0532 | 0.4212 | 0.0597 | 0. 0493 |
| 20ng | 0. 1388 | 0. 0553 | 0.1164 | 0.0578 | 0.0476 |
| 2ng | 0. 0507 | 0.0502 | 0.0603 | 0. 0584 | 0.0474 |

(continued)

| JYB14 | | | | | |
|---|---|---|---|---|---|
| Amount of antibody added | Human CD79B antigen | Mouse CD79B antigen | Human CD79A+CD79B antigen | Mouse CD79A+CD79B antigen | BSA |
| 200pg | 0.0501 | 0. 0569 | 0.0561 | 0. 0609 | 0. 0489 |

As can be seen from Tables 3-7, the anti-CD79B single-domain antibodies JYB4, JYB10, JYB8, JYB11, and JYB14 could all effectively and specifically bind to the human CD79B antigen, but failed to effectively bind to the mouse CD79B antigen.

The amino acid sequence of the anti-CD79B single-domain antibody JYB4 is set forth in SEQ ID NO:1. The anti-CD79B single-domain antibody JYB4 comprises CDR-H1, CDR-H2, and CDR-H3. The amino acid sequence of the CDR-H1 is set forth in SEQ ID NO:3, the amino acid sequence of the CDR-H2 is set forth in SEQ ID NO:5, and the amino acid sequence of the CDR-H3 is set forth in SEQ ID NO:7.

The amino acid sequence of the anti-CD79B single-domain antibody JYB10 is set forth in SEQ ID NO:9. The anti-CD79B single-domain antibody JYB10 comprises CDR-H1, CDR-H2, and CDR-H3. The amino acid sequence of the CDR-H1 is set forth in SEQ ID NO:11, the amino acid sequence of the CDR-H2 is set forth in SEQ ID NO:13, and the amino acid sequence of the CDR-H3 is set forth in SEQ ID NO:15.

The amino acid sequence of the anti-CD79B single-domain antibody JYB8 is set forth in SEQ ID NO:17. The anti-CD79B single-domain antibody JYB8 comprises CDR-H1, CDR-H2, and CDR-H3. The amino acid sequence of the CDR-H1 is set forth in SEQ ID NO:19, the amino acid sequence of the CDR-H2 is set forth in SEQ ID NO:20, and the amino acid sequence of the CDR-H3 is set forth in SEQ ID NO:22.

The amino acid sequence of the anti-CD79B single-domain antibody JYB11 is set forth in SEQ ID NO:23. The anti-CD79B single-domain antibody JYB11 comprises CDR-H1, CDR-H2, and CDR-H3. The amino acid sequence of the CDR-H1 is set forth in SEQ ID NO:24, the amino acid sequence of the CDR-H2 is set forth in SEQ ID NO:20, and the amino acid sequence of the CDR-H3 is set forth in SEQ ID NO:26.

The amino acid sequence of the anti-CD79B single-domain antibody JYB14 is set forth in SEQ ID NO:27. The anti-CD79B single-domain antibody JYB14 comprises CDR-H1, CDR-H2, and CDR-H3. The amino acid sequence of the CDR-H1 is set forth in SEQ ID NO:29, the amino acid sequence of the CDR-H2 is set forth in SEQ ID NO:20, and the amino acid sequence of the CDR-H3 is set forth in SEQ ID NO:31.

E. Flow Cytometry Assay

The CD79B-GFP molecule was designed, and the nucleic acid sequence encoding the CD79B-GFP molecule contained, from the 5' end to the 3' end, a nucleic acid sequence encoding the human CD79B antigen (a full-length protein containing signal peptide, UniProt ID: P40259), a nucleic acid sequence encoding the F2A peptide (self-cleaving peptide), and a nucleic acid sequence encoding the GFP (Green Fluorescent Protein) molecule. A plasmid containing the nucleic acid sequence encoding the CD79B-GFP molecule (CD79B-GFP plasmid) was constructed. The CD79B-GFP plasmid was transfected into HEK-293T cells using PEI reagent to overexpress the CD79B-GFP molecule. The method for constructing the CD79B-GFP plasmid is well known to those skilled in the art.

HEK-293T cells overexpressing the CD79B-GFP molecule were digested and divided into five groups.

In the present example, when synthesizing the anti-CD79B single-domain antibodies JYB4, JYB10, JYB8, JYB11, and JYB14, a nucleic acid sequence encoding the His-tag protein was added to the 3' end of the nucleic acid sequence encoding each of the anti-CD79B single-domain antibodies, so that the His-tag protein could be expressed along with each of the anti-CD79B single-domain antibodies.

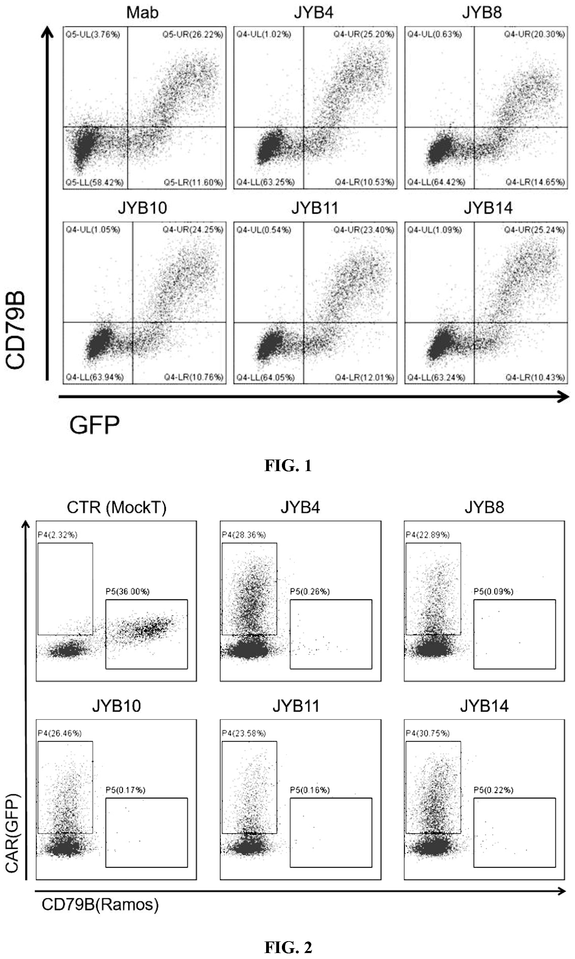

The digested HEK-293T cells of each group were respectively co-incubated with the anti-CD79B single-domain antibodies JYB4, JYB10, JYB8, JYB11, and JYB14 carrying His-tag protein at the end (each at an antibody concentration of 0.1 mg/mL) at 4 °C for 30 min, followed by incubation with APC-His tag antibody (Brand: BIOLEGEND, Cat. No.: #362605) at 4 °C for 30 min. In the control group, the digested HEK-293T cells were co-incubated with the commercially available anti-CD79B monoclonal antibody (Brand: BIOLEGEND, Cat. No.: #341404, fluorescence-labeled). On day 2, the cells of each group were detected by flow cytometry, respectively, and the results were shown in FIG. 1.

As can be seen from FIG. 1, the anti-CD79B single-domain antibodies JYB4, JYB10, JYB8, JYB11, and JYB14 exhibit comparable specific binding ability to the human CD79B antigen to the commercially available anti-CD79B antibody (monoclonal antibody) which has high specificity and strong affinity.

Example 2

EP 4 768 505 A1

Detection of CAR-T Cell Killing Ability

A. Construction of CD79B-CAR

The CD79B-targeting chimeric antigen receptor (CD79B-CAR) was designed. The CD79B-CAR comprised, from N-terminus to C-terminus, an extracellular antigen-binding region, a CD8α hinge region, a CD8α transmembrane region, a 4-1BB co-stimulatory signaling domain, and a CD3ζ intracellular signaling domain in sequence.

The extracellular antigen-binding region is the anti-CD79B single-domain antibody JYB4, JYB10, JYB8, JYB11, or JYB14.

The 5' end of the nucleic acid sequence encoding the CD79B-CAR is operably linked to the 3' end of the nucleic acid sequence encoding the CD8α signal peptide, enabling membrane expression of the CD79B-CAR on the cell surface by the action of the CD8α signal peptide.

To efficiently detect the expression of each CD79B-CAR, the nucleic acid sequence encoding the F2A peptide was linked to the 3' end of the nucleic acid sequence encoding each CD79B-CAR, and the nucleic acid sequence encoding the GFP molecule was linked to the 3' end of the nucleic acid sequence encoding the F2A peptide, thus constructing the CD79B-CAR-GFP molecule.

(1) The amino acid sequence of the CD8α signal peptide is set forth in SEQ ID NO:32.
(2) The extracellular antigen binding region is the anti-CD79B single-domain antibody JYB4, JYB10, JYB8, JYB11, or JYB14; the amino acid sequences of the anti-CD79B single-domain antibodies JYB4, JYB10, JYB8, JYB11, and JYB14 are set forth in SEQ ID NO:1, SEQ ID NO:9, SEQ ID NO:17, SEQ ID NO:23, and SEQ ID NO:27, respectively.
(3) The amino acid sequence of the hinge region of the CD8α is set forth in SEQ ID NO:33.
(4) The amino acid sequence of the transmembrane region of the CD8α is set forth in SEQ ID NO:34.
(5) The amino acid sequence of the 4-1BB co-stimulatory domain is set forth in SEQ ID NO:35.
(6) The amino acid sequence of the intracellular signaling domain of the CD3ζ is set forth in SEQ ID NO:36.
(7) The amino acid sequence of the F2A peptide is set forth in SEQ ID NO:37.
(8) The amino acid sequence of the GFP is set forth in SEQ ID NO:38.

The specific amino acid sequences of each domain of the CD79B-CAR are shown in Table 8 below.

Table 8

| SEQ ID NO:32 | MALPVTALLLPLALLLHAARP |
|---|---|
| SEQ ID NO:33 | TTTPAPRPPTPAPTIASQPLSLRPEACRPAAGGAVHTRGLDFACD |
| SEQ ID NO:34 | IYIWAPLAGTCGVLLLSLVITLYC |
| SEQ ID NO:35 | KRGRKKLLYIFKQPFMRPVQTTQEEDGCSCRFPEEEEGGCEL |
| | |
| SEQ ID NO:36 | RVKFSRSADAPAYQQGQNQLYNELNLGRREEYDVLDKRRGRDPEM GGKPRRKNPQEGLYNELQKDKMAEAYSEIGMKGERRRGKGHDGLY QGLSTATKDTYDALHMQALPPR |

B. Lentiviral Vector Packaging

The following four plasmids were prepared: pMD2.G envelope plasmid (comprising gene encoding wild-type VSV-G), pMDLg/pRRE packaging plasmid, pRSV-REV packaging plasmid, and main plasmid carrying nucleic acid encoding the CD79B-CAR-GFP and lentiviral backbone genome (the main plasmid was synthesized by conventional molecular cloning methods). The four plasmids were transfected into the HEK-293T cells via the PEI reagent to package lentiviral vectors CD79B-JYB4, CD79B-JYB10, CD79B-JYB8, CD79B-JYB11, and CD79B-JYB14 carrying nucleic acid sequences of CARs containing different anti-CD79B single-domain antibodies. The specific packaging steps are as follows:

9 μg of the main plasmid, 4 μg of the pMDLg/pRRE packaging plasmid, 2 μg of the pRSV-REV packaging plasmid, and 2 μg of the pMD2.G envelope plasmid were added into 1 mL of Opti-MEM alpha reduced-serum medium. After shaking well, 64 μL of PEI reagent was added. The mixture was gently pipetted to homogeneity and then allowed to

27

stand for 10 minutes before being added into the HEK-293T cell culture medium. The medium was replaced after 6 hours. The culture supernatant was collected at 48 hours post-transfection, followed by filtration through a 0.45 $\mu$m filter membrane and centrifugation at 50000 g for 2.5 hours. The supernatant was aspirated and discarded, and the lentiviral vectors were resuspended in 200 $\mu$L of F12 medium and cryopreserved at -80 °C.

Opti-MEM alpha reduced-serum medium, Brand: GIBCO, Cat. No.: #SP0272;
HEK-293T cell culture medium: DMEM + 10% FBS; DMEM: Brand: GIBCO, Cat. No.: #C12430500BT; FBS: Brand: EXCELL, Cat. No.: #FSP500;
F12 medium: Brand: GIBCO, Cat. No.: #C11330500BT;
Syringe filter: Brand: SORFA, Cat. No.: #622120.

C. <u>Infection of Activated T Cells (MOI = 5)</u>

Day 0: $1\times10^7$ human resting PBMCs were taken for each of 5 groups, and the PBMCs were adjusted to a cell concentration of $3\times10^7$ cells/mL using XVT medium + 1000 IU/mL IL-2. Anti-CD3 antibody was added to a final concentration of 1 $\mu$g/mL and anti-CD28 antibody was added to a final concentration of 2 $\mu$g/mL to stimulate and activate T cells (Mock T cells).

On Day 1, the original culture medium of each T cell culture system was removed by washing, and XVT medium + 1000 IU/mL IL-2 was added to adjust the cell concentration to $4\times10^6$ cells/mL. The lentiviral vectors CD79B-JYB4, CD79B-JYB10, CD79B-JYB8, CD79B-JYB11, and CD79B-JYB14 were added into each T cell culture system at MOI = 5 to prepare the CAR-T-JYB4 cells, CAR-T-JYB10 cells, CAR-T-JYB8 cells, CAR-T-JYB11 cells, and CAR-T-JYB14 cells, respectively.

On Day 2, the original culture medium of each T cell culture system was removed by washing, and the cell concentration of each group was adjusted to $1\times10^6$ to $2\times10^6$ cells/mL with XVT medium + 1000 IU/mL IL-2.

On Day 8, $3\times10^5$ cells of each of the CAR-T-JYB4 cells, CAR-T-JYB10 cells, CAR-T-JYB8 cells, CAR-T-JYB 11 cells, and CAR-T-JYB 14 cells prepared from each T cell culture system were taken respectively, and added into $1\times10^5$ CD79B$^+$ target Ramos cells (human B lymphoma cells) at E:T = 3:1 (effector-to-target ratio) for the killing assay. Control group was Mock T cell group (activated T cells). On Day 10, each group was detected by flow cytometry, and results were shown in FIG. 2.

XVT medium: Brand: IRVINE (Fujifilm), Cat. No.: #91154.

IL-2: Brand: Beijing Sihuan, Cat. No.: National Medical Product Approval No. S20040008.

Anti-CD3 antibody: Brand: BIOLEGEND, Cat. No.: #317354.

Anti-CD28 antibody: Brand: INVITROGEN, Cat. No.: #16-0289-81.

As can be seen from FIG. 2, the CAR-T-JYB4 cells, CAR-T-JYB10 cells, CAR-T-JYB8 cells, CAR-T-JYBI11 cells, and CAR-T-JYB14 cells could all effectively kill target Ramos cells.

Antibody used for the flow cytometry: Brand: BD, Cat. No.: #550955.

**Claims**

1. An anti-CD79B single-domain antibody, **characterized in that** the anti-CD79B single-domain antibody comprises CDR-H1, CDR-H2, and CDR-H3;

    wherein the CDR-H1 comprises an amino acid sequence as set forth in SEQ ID NO:3, the CDR-H2 comprises an amino acid sequence as set forth in SEQ ID NO:5, and the CDR-H3 comprises an amino acid sequence as set forth in SEQ ID NO:7; or
    wherein the CDR-H1 comprises an amino acid sequence as set forth in SEQ ID NO:11, the CDR-H2 comprises an amino acid sequence as set forth in SEQ ID NO:13, and the CDR-H3 comprises an amino acid sequence as set forth in SEQ ID NO:15; or
    wherein the CDR-H1 comprises an amino acid sequence as set forth in SEQ ID NO:19, the CDR-H2 comprises an amino acid sequence as set forth in SEQ ID NO:20, and the CDR-H3 comprises an amino acid sequence as set forth in SEQ ID NO:22; or
    wherein the CDR-H1 comprises an amino acid sequence as set forth in SEQ ID NO:24, the CDR-H2 comprises an amino acid sequence as set forth in SEQ ID NO:20, and the CDR-H3 comprises an amino acid sequence as set forth in SEQ ID NO:26; or
    wherein the CDR-H1 comprises an amino acid sequence as set forth in SEQ ID NO:29, the CDR-H2 comprises an amino acid sequence as set forth in SEQ ID NO:20, and the CDR-H3 comprises an amino acid sequence as set forth in SEQ ID NO:31.

2. The anti-CD79B single-domain antibody according to claim 1, **characterized in that** the anti-CD79B single-domain antibody further comprises FR1, FR2, FR3, and FR4;

wherein the FR1 comprises an amino acid sequence as set forth in SEQ ID NO:2, the FR2 comprises an amino acid sequence as set forth in SEQ ID NO:4, the FR3 comprises an amino acid sequence as set forth in SEQ ID NO:6, and the FR4 comprises an amino acid sequence as set forth in SEQ ID NO:8; or
wherein the FR1 comprises an amino acid sequence as set forth in SEQ ID NO:10, the FR2 comprises an amino acid sequence as set forth in SEQ ID NO:12, the FR3 comprises an amino acid sequence as set forth in SEQ ID NO:14, and the FR4 comprises an amino acid sequence as set forth in SEQ ID NO:16; or
wherein the FR1 comprises an amino acid sequence as set forth in SEQ ID NO:18, the FR2 comprises an amino acid sequence as set forth in SEQ ID NO:12, the FR3 comprises an amino acid sequence as set forth in SEQ ID NO:21, and the FR4 comprises an amino acid sequence as set forth in SEQ ID NO:16; or
wherein the FR1 comprises an amino acid sequence as set forth in SEQ ID NO:10, the FR2 comprises an amino acid sequence as set forth in SEQ ID NO:12, the FR3 comprises an amino acid sequence as set forth in SEQ ID NO:25, and the FR4 comprises an amino acid sequence as set forth in SEQ ID NO:16; or
wherein the FR1 comprises an amino acid sequence as set forth in SEQ ID NO:28, the FR2 comprises an amino acid sequence as set forth in SEQ ID NO:12, the FR3 comprises an amino acid sequence as set forth in SEQ ID NO:30, and the FR4 comprises an amino acid sequence as set forth in SEQ ID NO:16.

3. The anti-CD79B single-domain antibody according to claim 1 or 2, **characterized in that** the anti-CD79B single-domain antibody comprises an amino acid sequence as set forth in SEQ ID NO:1, SEQ ID NO:9, SEQ ID NO:17, SEQ ID NO:23 or SEQ ID NO:27, or an amino acid sequence having at least about 75%, 80%, 85%, 90%, 91%, 92%, 93%, 94%, 95%, 96%, 97%, 98%, 99% or 99.5% identity to the amino acid sequence as set forth in SEQ ID NO:1, SEQ ID NO:9, SEQ ID NO:17, SEQ ID NO:23 or SEQ ID NO:27.

4. An isolated nucleic acid, **characterized in that** the nucleic acid encodes the anti-CD79B single-domain antibody according to any one of claims 1 to 3.

5. A chimeric antigen receptor, **characterized in that** the chimeric antigen receptor comprises:

(a) an extracellular antigen-binding region;
(b) a transmembrane region; and
(c) an intracellular signaling domain;

wherein the extracellular antigen-binding region comprises the anti-CD79B single-domain antibody according to any one of claims 1 to 3.

6. The chimeric antigen receptor according to claim 5, **characterized in that** the transmembrane region is derived from a transmembrane region of at least one of following proteins: CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcεRIγ, IL7, IL12, IL15, SLAM, KIR2DL4, KIR2DS1, KIR2DS2, NKG2C, NKG2D, and CS1;
preferably, the transmembrane region is derived from the transmembrane region of CD8α.

7. The chimeric antigen receptor according to claim 5 or 6, **characterized in that** the intracellular signaling domain is derived from an intracellular signaling domain of at least one of following proteins: CD3ε, CD3γ, CD3δ, CD3ζ, CD79a, CD79b, FcεRIγ, FcεRβ, FcγRIIa, bovine leukemia virus gp30, Epstein-Barr virus (EBV) LMP2A, simian immunodeficiency virus PBj14 Nef, DAP10, DAP12, and intracellular signaling domains of other proteins whose intracellular signaling domain comprises at least one ITAM;
preferably, the intracellular signaling domain is derived from the intracellular signaling domain of CD3ζ.

8. The chimeric antigen receptor according to any one of claims 5 to 7, **characterized in that** the chimeric antigen receptor further comprises a hinge region, wherein the hinge region links the extracellular antigen-binding region and the transmembrane region;

preferably, the hinge region is derived from a hinge region of at least one of following proteins: CD28, CD8, CD8α, CD8β, CD3, CD45, Ig4, CD4, CD5, CD8, CD9, CD16, CD22, CD33, CD37, CD134, CD137, ICOS, and CD154;

more preferably, the hinge region is derived from the hinge region of CD8α.

9. The chimeric antigen receptor according to any one of claims 5 to 8, **characterized in that** the chimeric antigen receptor further comprises a costimulatory signaling domain;

preferably, the costimulatory signaling domain comprises a costimulatory signaling domain of at least one of following proteins: CD28, 4-1BB, CD27, CD2, CD7, CD8, CD8α, CD8β, OX40, CD226, DR3, SLAM, CDS, ICAM-1, NKG2D, NKG2C, B7-H3, 2B4, FcαRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88;
more preferably, the costimulatory signaling domain comprises the costimulatory signaling domain of 4-1BB.

10. The chimeric antigen receptor according to any one of claims 5 to 9, **characterized in that** the chimeric antigen receptor further comprises a signal peptide at N-terminus of the chimeric antigen receptor;

preferably, the signal peptide is derived from a signal peptide of at least one of following proteins: HLA-A, CD8α, CD33, Igκ, IL-2, and GM-CSFRα;
more preferably, the signal peptide is derived from the signal peptide of CD8α.

11. An isolated nucleic acid, **characterized in that** the nucleic acid encodes the chimeric antigen receptor according to any one of claims 5 to 10.

12. A vector, **characterized in that** the vector comprises the isolated nucleic acid according to claim 11;

preferably, the vector is at least one selected from the group consisting of lipid nanoparticles, virus-like particles, adenovirus, adeno-associated virus, lentiviral vector, and retroviral vector;
more preferably, the vector is a lentiviral vector or a retroviral vector.

13. A targeting vector, **characterized in that** a surface of the targeting vector comprises a targeting molecule, and the targeting molecule comprises the anti-CD79B single-domain antibody according to any one of claims 1 to 3.

14. The targeting vector according to claim 13, **characterized in that** the targeting vector is a lentiviral vector or a retroviral vector, the targeting molecule further comprises a transmembrane region, and the anti-CD79B single-domain antibody is directly or indirectly linked to the transmembrane region and is exposed on a surface of the lentiviral vector or the retroviral vector;
preferably, the transmembrane region is derived from a transmembrane region of any one of following proteins:

CD2, CD3, TCR, CD4, CD5, CD7, CD8, CD8α, CD8β, CD9, CD16, CD22, CD27, CD28, CD28H, CD30, CD33, CD37, CD40, CD45, CD64, CD80, CD84, CD154, CD166, CD226, CD244, 4-1BB, OX40, ICOS, ICAM-1, CTLA-4, PD-1, LAG-3, GITR, HVEM, DAP10, DAP12, TIM-1, LIGHT, ICOS, OX40, 2B4, BTLA, DNAM-1, DR3, FcεRIγ, IL7, IL12, IL15, SLAM, KIR2DL4, KIR2DS1, KIR2DS2, NKG2C, NKG2D, and CS1;
more preferably, the transmembrane region is derived from the transmembrane region of CD8α.

15. The targeting vector according to claim 14, **characterized in that** the targeting molecule further comprises a linking domain, and the anti-CD79B single-domain antibody is indirectly linked to the transmembrane region via the linking domain;

preferably, the linking domain is selected from the group consisting of:

(i) an immunoglobulin hinge region, wherein the immunoglobulin hinge region is selected from wild-type or modified hinge regions of IgG1, IgG2, IgG3, IgG4, IgA, and IgD;
(ii) a hinge region, wherein the hinge region is selected from wild-type or modified hinge regions of proteins selected from the group consisting of: CD28, CD7, CD8, CD8α, CD8β, CD3, CD45, CD4, CD5, CD8, CD9, CD16, CD22, CD33, CD37, CD134, CD137, ICOS, and CD154;
(iii) all or part of an Fc domain, wherein the Fc domain is one or more domains selected from the group consisting of CH1 domain, CH2 domain, and CH3 domain; and
(iv) a stalk domain of a type II C-lectin, wherein the type II C-lectin is selected from stalk domains of CD23, CD69, CD72, CD94, NKG2A, and NKG2D;

more preferably, the linking domain is the hinge region of CD8$\alpha$.

16. An engineered immune effector cell, **characterized in that** the engineered immune effector cell comprises the chimeric antigen receptor according to any one of claims 5 to 10 or the nucleic acid according to claim 11;

preferably, the engineered immune effector cell is at least one selected from the group consisting of T cell, B cell, NK cell, macrophage, dendritic cell, and induced pluripotent stem cell;
more preferably, the engineered immune effector cell is an engineered T cell.

17. The engineered immune effector cell according to claim 16, **characterized in that** the engineered immune effector cell is the engineered T cell, and the engineered T cell expresses the chimeric antigen receptor.

18. A composition, **characterized in that** the composition comprises a pharmaceutically acceptable excipient or carrier and any one of following components: (a) the anti-CD79B single-domain antibody according to any one of claims 1 to 3, (b) the chimeric antigen receptor according to any one of claims 5 to 10, or (c) the engineered immune effector cell according to claim 16 or 17.

19. A method for treating a disease in a subject, **characterized by** comprising administering a therapeutically effective amount of the anti-CD79B single-domain antibody according to any one of claims 1 to 3, the engineered immune effector cell according to claim 16 or 17, or the composition according to claim 18 to the subject, wherein

preferably, the disease is a B cell-related disease and/or a CD79B-related disease;
more preferably, the disease is a B cell-related and/or CD79B-related malignancy;
even more preferably, the B cell-related malignancy is B cell leukemia or B cell lymphoma, wherein the B cell leukemia or the B cell lymphoma is selected from the group consisting of marginal zone lymphoma, diffuse large B-cell lymphoma, mantle cell lymphoma, primary central nervous system lymphoma, primary mediastinal B-cell lymphoma, small lymphocytic lymphoma, B-cell prolymphocytic leukemia, follicular lymphoma, Burkitt lymphoma, primary intraocular lymphoma, chronic lymphocytic leukemia, acute lymphoblastic leukemia, hairy cell leukemia, precursor B-cell lymphoblastic leukemia, non-Hodgkin lymphoma, high-grade B-cell lymphoma, and multiple myeloma.

20. Use of the anti-CD79B single-domain antibody according to any one of claims 1 to 3, the chimeric antigen receptor according to any one of claims 5 to 10, the vector according to claim 12, the engineered immune effector cell according to claim 16 or 17, or the composition according to claim 18 in manufacture of a medicament for treating a B cell-related and/or CD79B-related malignancy, a B cell-related and/or CD79B-related autoimmune and/or inflammatory disease; preferably, the B cell-related malignancy is B cell leukemia or B cell lymphoma, wherein the B cell leukemia or B cell lymphoma is selected from the group consisting of marginal zone lymphoma, diffuse large B-cell lymphoma, mantle cell lymphoma, primary central nervous system lymphoma, primary mediastinal B-cell lymphoma, small lymphocytic lymphoma, B-cell prolymphocytic leukemia, follicular lymphoma, Burkitt lymphoma, primary intraocular lymphoma, chronic lymphocytic leukemia, acute lymphoblastic leukemia, hairy cell leukemia, precursor B-cell lymphoblastic leukemia, non-Hodgkin lymphoma, high-grade B-cell lymphoma, and multiple myeloma.

21. A reagent for detecting a CD79B protein or a functional domain comprising epitopes of the CD79B protein, **characterized in that** the reagent comprises the anti-CD79B single-domain antibody according to any one of claims 1 to 3;

preferably, the CD79B protein is a human CD79B protein;
more preferably, the reagent further comprises a modification moiety linked to the anti-CD79B single-domain antibody, wherein the modification moiety comprises a detectable label or a therapeutic agent;
even more preferably, the detectable label comprises at least one selected from the group consisting of an enzyme, a tag protein, a radionuclide, a fluorescent dye, a luminescent substance, and biotin.

22. A method for detecting a CD79B protein or a functional domain comprising epitopes of the CD79B protein for non-diagnostic purposes, **characterized in that** the method comprises obtaining a sample suspected of containing the CD79B protein or the functional domain comprising the epitopes of the CD79B protein, contacting the anti-CD79B single-domain antibody according to any one of claims 1 to 3 or the reagent according to claim 21 with the sample to form an antibody-antigen complex, and detecting presence of the antibody-antigen complex;
preferably, the CD79B protein is a human CD79B protein.

**FIG. 1**

**FIG. 2**

## INTERNATIONAL SEARCH REPORT

| International application No. |
| --- |
| **PCT/CN2024/121623** |

| | |
| --- | --- |
| **A. CLASSIFICATION OF SUBJECT MATTER** | |

C07K16/28(2006.01)i; C07K19/00(2006.01)i; C12N15/62(2006.01)i; C12N5/10(2006.01)i; A61K39/00(2006.01)i; A61P35/00(2006.01)i

According to International Patent Classification (IPC) or to both national classification and IPC

| | |
| --- | --- |
| **B. FIELDS SEARCHED** | |

Minimum documentation searched (classification system followed by classification symbols)

IPC: C07K, C12N, A61K, A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, NCBI, 中文专利序列检索系统, National Sequence Database of Chinese Patent, genbank, EMBL-EBI, 深圳市济因生物科技有限公司, 黄可, CD79B, 单域抗体, 单结构域抗体, 纳米抗体, single domain antibody, sdAb, nanobody, VHH, 嵌合抗原受体, CAR, chimeric antigen receptor, SEQ ID NOs: 1-31

**C. DOCUMENTS CONSIDERED TO BE RELEVANT**

| Category* | Citation of document, with indication, where appropriate, of the relevant passages | Relevant to claim No. |
| --- | --- | --- |
| A | WO 2021222944 A1 (BOARD OF REGENTS, THE UNIVERSITY OF TEXAS SYSTEM) 04 November 2021 (2021-11-04) abstract, and claims 1-92 | 1-22 |
| A | WO 2019241688 A1 (BLUEBIRD BIO, INC.) 19 December 2019 (2019-12-19) entire document | 1-22 |
| A | CN 115772222 A (CARBIOGENE THERAPEUTICS CO., LTD.) 10 March 2023 (2023-03-10) entire document | 1-22 |
| A | CN 109265565 A (SOOCHOW UNIVERSITY) 25 January 2019 (2019-01-25) entire document | 1-22 |

| ✓ Further documents are listed in the continuation of Box C. | ✓ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **27 November 2024** | **26 December 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2024/121623** |

| C. | DOCUMENTS CONSIDERED TO BE RELEVANT | |
|---|---|---|
| Category* | Citation of document, with indication, where appropriate, of the relevant passages | Relevant to claim No. |
| A | 刘伯宁 等 (LIU, Boning et al.). "关于嵌合抗原受体修饰T细胞产品药学评价的思考 (Regulatory Perspective for Chemistry Manufacturing and Controls Considerations in Chimeric Antigen Receptor-Modified T Cell Therapy)" 药学学报 (Acta Pharmaceutica Sinica), 31 December 2018 (2018-12-31), pages 1637-1644 | 1-22 |
| A | 周祺祺 等 (ZHOU, Qiqi et al.). "嵌合抗原受体T细胞制备及临床应用的影响因素研究进展 (Non-official translation: Research Progress on Influencing Factors in Preparation and Clinical Application of Chimeric Antigen Receptor T Cells)" 山东医药 (Shandong Medical Journal), Vol. 59, No. (36), 31 December 2019 (2019-12-31), pp. 104-107 | 1-22 |

Form PCT/ISA/210 (second sheet) (July 2022)

**INTERNATIONAL SEARCH REPORT**

International application No.

**PCT/CN2024/121623**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2024/121623**

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **19**
   because they relate to subject matter not required to be searched by this Authority, namely:

   Claim 19 relates to a method for treating a disease as defined in PCT Rule 39.1(iv); however, a search opinion is still provided on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/121623**

| Patent document cited in search report | | | Publication date (day/month/year) | Patent family member(s) | | | Publication date (day/month/year) |
|---|---|---|---|---|---|---|---|
| WO | 2021222944 | A1 | 04 November 2021 | US | 2023183371 | A1 | 15 June 2023 |
| | | | | CA | 3181566 | A1 | 04 November 2021 |
| | | | | JP | 2023525496 | A | 16 June 2023 |
| | | | | AU | 2021264094 | A1 | 24 November 2022 |
| | | | | KR | 20230003580 | A | 06 January 2023 |
| | | | | EP | 4142778 | A1 | 08 March 2023 |
| | | | | EP | 4142778 | A4 | 05 June 2024 |
| WO | 2019241688 | A1 | 19 December 2019 | US | 2022226374 | A1 | 21 July 2022 |
| | | | | EP | 3806857 | A1 | 21 April 2021 |
| | | | | EP | 3806857 | A4 | 02 March 2022 |
| CN | 115772222 | A | 10 March 2023 | None | | | |
| CN | 109265565 | A | 25 January 2019 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 199634103 A
- US 6013516 A
- US 5994136 A

### Non-patent literature cited in the description

- **DANIEL P. STIES** ; **ABBA I. TERR** ; **TRISTRAM G. PARSOLW**. Basic and Clinical Immunology. Appleton & Lange, 1994, 71
- **KARLIN** ; **ALTSCHUL**. *Proc. Natl. Acad. Sci. USA*, 1990, vol. 87, 2264-2268
- **ALTSCHUL et al.** *J. Mol. Biol.*, 1990, vol. 215, 403-410
- **KARLIN** ; **ALTSCHUL**. *Proc. Natl. Acad. Sci. USA*, 1993, vol. 90, 5873-5877
- **ALTSCHUL et al.** *Nucleic Acids Res.*, 1997, vol. 25, 3389-3402
- **CHEN X** ; **ZARO JL** ; **SHEN WC**. Fusion protein linkers: property, design and functionality. *Adv Drug Deliv Rev.*, October 2013, vol. 65 (10), 1357-69
- **ARGOS P.** An investigation of oligopeptides linking domains in protein tertiary structures and possible candidates for general gene fusion.. *J Mol Biol.*, 1990, vol. 211, 943-958
- **CHEN X** ; **ZARO JL** ; **SHEN WC.** Fusion protein linkers: property, design and functionality. *Adv Drug Deliv Rev.*, October 2013, vol. 65 (10), 1357-69
- **SAMBROOK et al.** Molecular Cloning: A Laboratory Manual. Cold Spring Harbor Laboratory Press, 2001
- **MICHAEL C. MILONE et al.** *Molecular Therapy*, 2009, vol. 17 (8), 1453-1464
- **OWJI et al.** A comprehensive review of signal peptides: Structure, roles, and applications. *European Journal of Cell Biology.*, 2018, vol. 97 (6), 422-441
- **BLOBEL G** ; **DOBBERSTEIN B et al.** Transfer of proteins across membranes. I. Presence of proteolytically processed and unprocessed nascent immunoglobulin light chains on membrane-bound ribosomes of murine myeloma. *The Journal of Cell Biology*, 1975, vol. 67 (3), 835-51
- **RAPOPORT TA**. Protein translocation across the eukaryotic endoplasmic reticulum and bacterial plasma membranes. *Nature*, 2007, vol. 450 (7170), 663-9
- **MERTEN OW et al.** Production of lentiviral vectors. *Mol Ther Methods Clin Dev.*, 2016, vol. 3, 16017
- **DULL et al.** *J. Virol.*, 1998, vol. 72, 8463-71
- **MIYOSHI et al.** *J. Virol.*, 1998, vol. 72, 8150-57
- **NALDINI et al.** *Science*, 1996, vol. 272, 263-7
- **ZUFFEREY et al.** *J. Virol.*, 1998, vol. 72, 9873-9880
- **DULL et al.** *J. Virol.*, 1998, vol. 72, 8463-8471
- **SAMBROOK, J.** ; **FRITSCH, E.F.** ; **MANIATIS, T.** Molecular Cloning: A Laboratory Manual. Cold Spring Harbor Laboratory Press, 2001
- **PETER M RABINOVICH**. *Human Gene Therapy*, 2006, vol. 17, 1027-1035
- **KUMIKO UI-TEI**. *FEBS Letters*, 2000, vol. 479, 79-82
- **LIEBERMAN**. *Pharmaceutical Dosage Forms*, 1992, vol. 1-3
- **LLOYD**. *The Art, Science and Technology of Pharmaceutical Compounding*, 1999
- **PICKAR**. *Dosage Calculations*, 1999